# EUROPÄISCHE PATENTSCHRIFT

(11) **EP 3 877 501 B1**
(45) Veröffentlichungstag und Bekanntmachung des Hinweises auf die Patenterteilung: **21.08.2024**
(21) Anmeldenummer: 20702785.5
(22) Anmeldetag: 30.01.2020
(51) Int. Cl.: C12M 1/12, B01J 19/00, C08G 83/00, C08L 101/00, C12M 1/00, B01L 3/00

(54) **GEMUSTERTE, DENDRIMERE SUBSTRATOBERFLÄCHEN SOWIE DEREN HERSTELLUNG UND VERWENDUNG**
PATTERNED, DENDRIMERIC SUBSTRATE SURFACES AND PRODUCTION AND USE THEREOF
SURFACES DE SUBSTRAT DENDRIMÈRE À MOTIFS, AINSI QUE LEUR FABRICATION ET LEUR UTILISATION

(30) Priorität: 15.02.2019 DE 102019001137
(43) Veröffentlichungstag der Anmeldung: 15.09.2021
(73) Patentinhaber: Karlsruher Institut für Technologie, 76131 Karlsruhe (DE)
(72) Erfinder: BENZ, Maximilian, 76646 Bruchsal (DE); LEVKIN, Pavel A., 76344 Eggenstein-Leopoldshafen (DE)
(74) Vertreter: Müller-Boré & Partner Patentanwälte PartG mbB
(86) Internationale Anmeldenummer: PCT/EP2020/052235
(87) Internationale Veröffentlichungsnummer: WO 2020/164917

(56) Entgegenhaltungen:
- US-A- 6 077 500
- US-A1- 2003 099 930
- CAMINADE ET AL: "Uses of dendrimers for DNA microarrays", SENSORS, MDPI AG, CH, vol. 6, 24 August 2006 (2006-08-24), pages 901 - 914, XP002694383, ISSN: 1424-8220, DOI: 10.3390/S6080901
- KIM M H ET AL: "Designing culture surfaces based on cell anchoring mechanisms to regulate cell morphologies and functions", BIOTECHNOLOGY ADVANCES, ELSEVIER PUBLISHING, BARKING, GB, vol. 28, no. 1, 1 January 2010 (2010-01-01), pages 7 - 16, XP026795997, ISSN: 0734-9750, [retrieved on 20090815]

## Beschreibung

Die vorliegende Erfindung betrifft ein gemustertes Substrat umfassend erste Bereiche mit ersten Dendrimerstrukturen und zweite Bereiche mit zweiten Dendrimerstrukturen auf einer Oberfläche des Substrats sowie ein Verfahren zur Herstellung eines gemusterten Substrats und die Verwendung eines gemusterten Substrats für die chemische Synthese eines chemischen Syntheseproduktes, als Charakterisierungsplattform und/oder als Plattform zur Zellbehandlung und/oder Zellkultivierung.

Die Entwicklung neuer Wirkstoffe und biologisch interessanter Moleküle ist ein Zeitund Ressourcen-aufwändiger Prozess. Tausende neuer Verbindungen müssen zunächst einzeln hergestellt und charakterisiert werden, bevor sie in Hochdurchsatz-Screenings auf ihre biologische Aktivität und Eigenschaften untersucht werden können. Dabei finden sämtliche Stationen dieser Molekül-Entwicklung sowohl zeitlich als auch räumlich getrennt voneinander statt.

In der chemischen Synthese müssen zunächst tausende Verbindungen einzeln, nacheinander synthetisiert werden, um eine primäre Molekül-Bibliothek aufzubauen. Die Synthese findet meist in Rundkolben statt, wobei Reagenzien häufig im Milliliter/Milligramm-Maßstab eingesetzt werden. Die klassische organische Synthese findet dabei in Lösung statt, wobei fast immer organische Lösungsmittel (u.a. Ethanol, Aceton, Dichlormethan (DCM), Dimethylformamid (DMF), Dimethylsulfoxid (DMSO) etc.) verwendet werden. Anschließend müssen sämtliche Produkte charakterisiert werden, um Aussagen über den Syntheseerfolg treffen zu können. Hierfür genügen häufig zwar kleine Mengen der Verbindung, der Brennpunkt im Übergang zwischen chemischer Synthese und Charakterisierung liegt allerdings in der Wahl der Charakterisierungs-Methode und der damit verbundenen Geräte-spezifischen Infrastruktur.

Die Methode der Wahl zur Charakterisierung großer Molekülbibliotheken stellt die Matrix-Assistierte Laser-Desorption-Ionisierung mit Flugzeitanalyse (MALDI-TOF) Massenspektrometrie (MS) dar. MALDI-TOF MS benötigt zum einen nur sehr wenig Probensubstanz, zum anderen handelt es sich hierbei um eine sehr schnelle, einfach zu automatisierende Methode, mit der in kürzester Zeit große Molekül-Bibliotheken charakterisiert werden können.

Allerdings benötigt MALDI-TOF MS eine spezielle Plattform, deren Oberfläche elektrisch leitfähig ist. Daher müssen zunächst sämtliche zuvor synthetisierten Verbindungen der Molekül-Bibliothek einzeln nacheinander auf die MALDI-TOF-Plattform übertragen werden. Bei diesem zeitaufwändigen Vorgang fällt ein immenser Betrag an Verbrauchsmaterialien, wie beispielsweise Pipettenspitzen (pro Verbindung eine Spitze zum Übertragen der Verbindung und eine weitere zum Auftragen von Matrix-Lösung, die zur Analyse benötigt wird), an.

Eine noch größere Herausforderung stellt das nachfolgende zellbiologische Screening dar. Während die Molekül-Bibliothek noch unter harschen Bedingungen (hohe Temperaturen, organische Lösungsmittel, Schutzgasatmosphäre etc.) hergestellt werden kann, können Zellexperimente nur unter sehr milden Bedingungen (Raumtemperatur, wässrige Lösungsmittel, Sauerstoff-Kohlenstoffdioxid-Atmosphäre etc.) durchgeführt werden.

Aufgrund der räumlichen und zeitlichen Trennung, und aufgrund des immensen Aufwands an Personal, Chemikalien, Lösungsmittel, Zellsuspension, und anderen Verbrauchsmaterialien beträgt der Entwicklungszeitrahmen für ein einziges neues Medikament häufig über 20 Jahre und verbraucht ein Budget von mehr als 800 Millionen US-Dollar. Eine Zusammenführung unterschiedlicher Arbeitsabläufe, wie etwa die Synthese von Verbindungen der Molekül-Bibliothek und deren Charakterisierung und zellbiologisches Screening, könnte diesen Zeitrahmen verkürzen und zu einer Kostensenkung führen.

Prinzipiell könnte eine Synthese in miniaturisierter Form beispielsweise in einem Microarray-System erfolgen. Grundsätzlich gibt es zwei verschiedene Arten von Microarrays, die sich in der Art der gemusterten Oberflächen unterscheiden und die von einem Fachmann anhand des zu verwendenden Lösungsmittels entsprechend gewählt werden können. Für wässrige Lösungsmittel mit einer hohen Oberflächenspannung (mehr als 72.2 mN m⁻¹) werden allgemein *high surface tension liquids* (HSTL) Microarrays verwendet, wohingegen für organische Lösungsmittel mit einer niedrigeren Oberflächenspannung (weniger als 72.2 mN m⁻¹) *low surface tension liquids* (LSTL) Microarrays verwendet werden. Es ist jedoch nicht möglich eines dieser Arrays für die jeweils anderen Lösungsmittel zu verwenden. US 2003/099930 A1 beschreibt ein gemustertes Substrat umfassend erste Bereiche und zweite Bereiche auf einer Oberfläche des Substrats, wobei die ersten Bereiche Dendrimerstrukturen aufweisen.

Ferner war es bisher nicht möglich, eine kombinatorische Synthese in flüssiger Phase, als auch die Charakterisierung durch z.B. MALDI-TOF MS, und biologisches Screening im Tröpfchen-Array-Format auf einem einzigen Substrat in direkter Abfolge durchzuführen.

Somit liegt der vorliegenden Erfindung die Aufgabe zugrunde, ein Substrat bereitzustellen, welches es ermöglicht, eine kombinatorische Synthese in flüssiger Phase, als auch die Charakterisierung durch z.B. MALDI-TOF MS, und ein biologisches Screening im Tröpfchen-Array-Format auf diesem in direkter Abfolge durchzuführen und dabei unterschiedliche Lösungsmittel, insbesondere Lösungsmittel mit unterschiedlichen Oberflächenspannungen, zuzulassen.

Diese Aufgabe wird durch die in den Ansprüchen gekennzeichneten Ausführungsformen gelöst.

Insbesondere betrifft die vorliegende Erfindung ein gemustertes Substrat umfassend erste Bereiche und zweite Bereiche auf einer Oberfläche des Substrats, wobei die ersten Bereiche erste Dendrimerstrukturen und die zweiten Bereiche zweite Dendrimerstrukturen aufweisen; die Dendrimerstrukturen jeweils kovalent mit der Substratoberfläche verbunden sind; die zweiten Bereiche die ersten Bereiche umschließen; die zweiten Dendrimerstrukturen mindestens ein Strukturelement aufweisen, das sich von den Strukturelementen der ersten Dendrimerstrukturen unterscheidet; und die Substratoberfläche Hydroxylgruppen oder Silanolgruppen aufweist.

Das erfindungsgemäße Substrat ermöglich es vorteilhafterweise eine flache Plattform bereitzustellen, auf der vorzugsweise klar definierte und voneinander abgegrenzte Tröpfchen sowohl mit organischen Lösungsmitteln mit geringer Oberflächenspannung für z.B. organische Flüssigphasen-Synthese als auch mit wässrigen Lösungsmitteln mit hoher Oberflächenspannung für z.B. biologische Hochdurchsatz-Screenings im Array-Format zu erzeugen. Durch das erfindungsgemäße Substrat ist er vorzugsweise ferner möglich eine kombinatorische Synthese in flüssiger Phase als auch die Charakterisierung durch bspw. MALDI-TOF MS und ein biologisches Screening im Tröpfchen-Array-Format auf einem einzigen Substrat in direkter Abfolge durchzuführen.

Insbesondere besitzt das erfindungsgemäße Substrat vorzugsweise eine Oberflächenbeschaffenheit, die kompatibel für MALDI-TOF MS ist und dabei keine Einschränkungen für den chemischen Syntheseprozess (bezüglich Stabilität der Oberfläche) und biologische Anwendung (in Hinsicht auf Zellkompatibilität) aufweist.

Durch das Vereinen, die Miniaturisierung und Parallelisierung sämtlicher Bereiche der frühen Phase der Medikamentenentwicklung (Synthese, Charakterisierung und Screening großer Molekül-Bibliotheken) auf einem einzigen Substrat ermöglicht das erfindungsgemäße Substrat vorzugsweise eine erhebliche Effizienzsteigerung in der Entwicklung neuer, biologisch interessanter Verbindungen, andererseits ermöglicht sie vorzugsweise aber auch eine drastische Kostensenkung dieser Entwicklung im Vergleich zu bestehenden Methoden.

Erfindungsgemäß ist das Substrat gemustert, besitzt also eine gemusterte Oberflächenschicht. Die gemusterte Oberflächenschicht wird durch die ersten Bereiche und die zweiten Bereiche auf mindestens einer Oberfläche des Substrats gebildet, wobei die zweiten Bereiche die ersten Bereiche (parallel zur Substratoberfläche) umschließen. Das Material des Substrats ist nicht besonders beschränkt, solange das Substrat eine Oberfläche mit Hydroxylgruppen oder Silanolgruppen aufweist. Ein solches Substrat kann direkt oder beispielsweise auch durch Aufbringen einer Beschichtung mit Hydroxylgruppen oder Silanolgruppen auf ein Grundmaterial ohne Hydroxylgruppen bereitgestellt werden. Vorzugsweise umfasst das Substrat Metalle mit einer oxidierten Oberfläche, wie bspw. Aluminium, vorzugsweise Aluminiumfolie, Halbleitermaterialien, Glas, Siliziumdioxid, Siliziumdioxid-Nanopartikel (engl. Silica nanoparticles), Indiumoxid, Zinnoxid, Mischoxide, Silikon, Polydimethylsiloxan (PDMS) oder ein organisches Polymer mit Oberflächenhydroxylgruppen wie Poly(2-hydroxyethylmethacrylat-co-ethylendimethacrylat) (HEMA-EDMA-Copolymer).

In einer bevorzugten Ausführungsform weist das gemusterte Substrat eine flache Oberfläche auf. Obwohl die Form des Substrats keinen besonderen Beschränkungen unterliegt, weist das Substrat vorzugsweise die Form eines (Objekt-)Trägers, eines Blechs, einer Schicht, einer Beschichtung oder eines Films, noch bevorzugter eines (Objekt-)Trägers (beschichtet oder unbeschichtet), auf. Dabei umfasst der Begriff "Folie" sowohl freistehende Folien als auch Folien, die auf einem anderen Substrat aufgebracht sind. In einer bevorzugten Ausführungsform der vorliegenden Anwendung weist das Substrat eine Oberfläche auf, die ausgewählt ist aus der Gruppe, bestehend aus Glasoberfläche, Halbleitermaterialien (wie bspw. Siliziumwafer und Indiumzinnoxid (ITO, engl. Indium tin oxide)), flacher Kunststofffolie, Metalloxidoberfläche und Metalloberfläche. Vorzugsweise weist die Oberfläche des Substrats, die die ersten und zweiten Bereiche aufweist, ein elektrisch leitfähiges Material auf bzw. besteht daraus. Vorzugsweise weist das Substrat eine Oberfläche aus Indiumzinnoxid auf. Besonders bevorzugt ist das Substrat ein Indiumzinnoxidbeschichtetes Glas. Die Bemusterung einer solchen Oberfläche erhält vorzugsweise die elektrische Leitfähigkeit der Oberfläche. Ein entsprechend gemustertes Substrat ist besonders vorteilhaft in Bezug auf die Verwendbarkeit bei Subtanzsynthese als auch direkter Charakterisierung der Substanzen bspw. mittels MALDI-TOF MS.

Erfindungsgemäß umfasst das gemusterte Substrat erste Bereiche und zweite Bereiche auf einer Oberfläche des Substrats. Die ersten Bereiche und zweiten Bereiche sind in ihrer Form nicht sonderlich eingeschränkt. Die ersten Bereiche können kreisförmig, sternförmig oder polygonal (dreieckig, rechteckig, fünfeckig, sechseckig, ...) sein und die zweiten Bereiche umschließen diese entsprechend. Erste Bereiche mit einer dreieckigen oder rechteckigen Form erlauben eine enge Anordnung der ersten Bereiche auf dem Substrat. Vorzugsweise bilden die ersten und zweiten Bereiche ein Microarray.

Auf Grund seiner speziellen Oberflächenmusterung ist das erfindungsgemäße gemusterte Substrat in der Lage auf dieser gemusterten Oberfläche mindestens einen Tropfen einer Lösung zu tragen. In einer bevorzugten Ausführungsform ist ein gemustertes Substrat dazu geeignet mindestens 1 Tropfen pro cm² der gemusterten Oberfläche zu tragen, mehr bevorzugt mindestens 4 Tropfen pro cm² der gemusterten Oberfläche, noch mehr bevorzugt mindestens 8 Tropfen pro cm² der gemusterten Oberfläche und am meisten bevorzugt mindestens 16 Tropfen pro cm² der gemusterten Oberfläche. Die obere Grenze der Anzahl der Tropfen pro cm² der gemusterten Oberfläche ist nicht sonderlich beschränkt und kann je nach Anforderung bestimmt werden. Entsprechende Tropfen, die auf der gemusterten Oberfläche eines gemusterten Substrats angeordnet werden können, haben beispielsweise ein Volumen von 100 pL bis 1 mL, vorzugsweise von 1 nL bis 100 µL, noch bevorzugter von 100 nL bis 50 µL und am meisten bevorzugt von 500 nL bis 25 µL.

Die ersten Bereiche können beispielsweise dreieckig oder rechteckig sein mit einer Seitenlänge von 5,0 mm oder weniger, 3,0 mm oder weniger, 1,0 mm oder weniger oder 500 µm oder weniger. In einer bevorzugten Ausführungsform der vorliegenden Erfindung sind die ersten Bereiche rund mit einem Durchmesser von 5,0 mm oder weniger, mehr bevorzugt 3,0 mm oder weniger, noch mehr bevorzugt 1,0 mm oder weniger. Die zweiten Bereiche, welche die ersten Bereiche umschließen, haben vorzugsweise eine Breite von 2,0 mm oder weniger, mehr bevorzugt 1,0 mm oder weniger und am meisten bevorzugt 0,5 mm oder weniger.

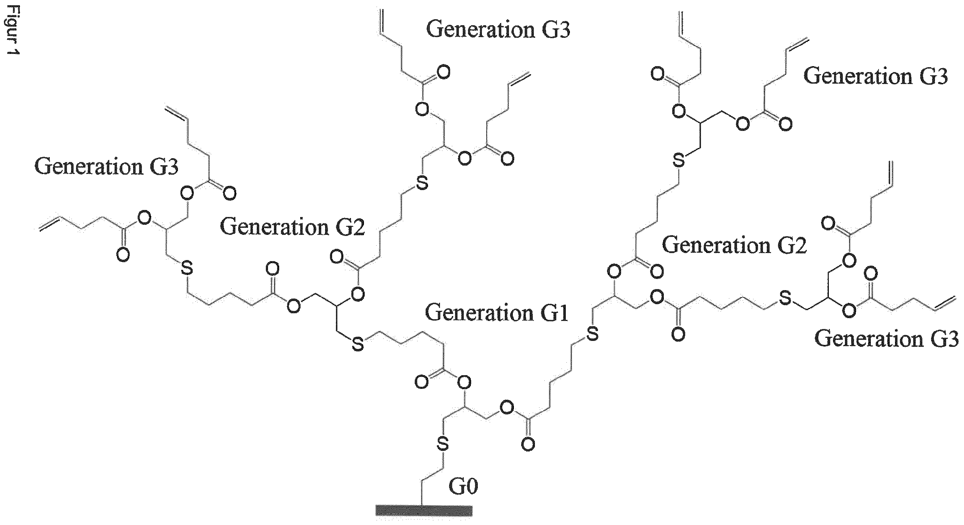

Erfindungsgemäß weisen die ersten Bereiche erste Dendrimerstrukturen und die zweiten Bereiche zweite Dendrimerstrukturen auf. Der Begriff "Dendrimerstrukturen" bezieht sich dabei auf Strukturen, die sich ausgehend von einem Kern verästeln (vgl. Figur 1). Dabei weisen die Dendrimerstrukturen Verästelungen von repetitiven Einheiten auf. Die repetitiven Einheiten weisen jeweils unabhängig voneinander mindestens zwei Verzweigungsstellen, vorzugsweise zwei bis fünf Verzweigungsstellen, noch bevorzugter zwei bis vier Verzweigungsstellen, noch bevorzugter zwei oder drei Verzweigungsstellen und am meisten bevorzugt zwei Verzweigungsstellen, auf. Die Dendrimerstrukturen weisen mindestens zwei Generationen an repetitiven Einheiten auf, d.h. ausgehend vom Kern weisen die Dendrimerstrukturen mindestens zwei aufeinanderfolgende repetitiven Einheiten (pro Hauptast) auf. Vorzugsweise weisen die Dendrimerstrukturen jeweils unabhängig voneinander zwei bis fünf, noch bevorzugter zwei bis vier, noch bevorzugter zwei oder drei, und am meisten bevorzugt drei Generationen an repetitiven Einheiten auf. Die Dendrimerstrukturen sind erfindungsgemäß jeweils kovalent mit der Substratoberfläche verbunden. Der Kern der Dendrimerstrukturen ist somit kovalent mit der Substratoberfläche verbunden. An ihren Enden weisen die Dendrimerstrukturen Endgruppen auf. Die Endgruppen können der letzten Generation an repetitiven Einheiten entsprechen oder unterschiedliche Strukturelemente (inkl. Teilstrukturen der repetitiven Einheiten) sein, die an die letzte Generation an repetitiven Einheiten anknüpfen.

Erfindungsgemäß weisen die zweiten Dendrimerstrukturen mindestens ein Strukturelement auf, das sich von den Strukturelementen der ersten Dendrimerstrukturen unterscheidet. Dabei kann es sich sowohl um die repetitiven Einheiten, den Kern und/oder die Endgruppen der Dendrimerstrukturen handeln. Vorzugsweise unterscheiden sich die ersten und zweiten Dendrimerstrukturen in den Endgruppen, besonders bevorzugt nur in den Endgruppen.

Durch das unterschiedliche Strukturelement ist es vorzugsweise möglich, derart unterschiedliche erste und zweite Bereiche zu bilden, dass sich in den ersten Bereichen jeweils klar definierte und voneinander abgegrenzte Tröpfchen, sowohl mit organischen Lösungsmittel mit geringer Oberflächenspannung für organische Flüssigphasen-Synthese als auch mit wässrigen Lösungsmittel mit hoher Oberflächenspannung, ausbilden können. Die ersten Bereiche werden dabei durch die sie umgebenden zweiten Bereiche voneinander abgegrenzt. Vorzugsweise sind die ersten Bereiche hydrophil, noch bevorzugter hydrophil und omniphil, und die zweiten Bereiche hydrophob, noch bevorzugter hydrophob und omniphob.

Im Allgemeinen können Oberflächen in Abhängigkeit ihres Kontaktwinkels mit Wasser als hydrophil oder hydrophob eingestuft werden. Eine Oberfläche mit einem statischen Kontaktwinkel von mindestens 90° wird hierbei als hydrophob bezeichnet, während eine Oberfläche mit einem statischen Kontaktwinkel von weniger als 90° als hydrophil bezeichnet wird. Es wird regelmäßig zwischen zwei verschiedenen Kontaktwinkeln unterschieden: einem statischen und einem dynamischen Kontaktwinkel. Statische Kontaktwinkel (θₛₜₐₜ) werden bestimmt, indem ein Tropfen auf eine Oberfläche gegeben wird und der Kontaktwinkel des in Ruhe aufliegenden Tropfens mit der Oberfläche mit einem Goniometer oder einer Kamera mit spezieller Software gemessen wird (*sessile drop* Messung). Dynamische Kontaktwinkel hingegen stellen Kontaktwinkel außerhalb eines Gleichgewichtszustandes dar und werden während des Fortschreitens (θ_{adv}) oder Rückzugs (θ_{rec}) eines Tropfens gemessen. Der Unterschied zwischen θ_{adv} und θ_{rec} wird als Kontaktwinkelhysterese bezeichnet.

In einer bevorzugten Ausführungsform der vorliegenden Erfindung haben hydrophobe Bereiche, vorzugsweise die zweiten Bereiche, einen statischen Wasser-Kontaktwinkel von mindestens 90°, bevorzugt mehr als 95°, noch bevorzugter mehr als 100°, noch bevorzugter mehr als 105° und am meisten bevorzugt mehr als 110°. Vorzugsweise weisen hydrophobe Bereiche, vorzugsweise die zweiten Bereiche, einen Wasser-Rückzugs-Kontaktwinkel (θ_{rec}) von mindestens 70°, bevorzugt mehr als 80°, noch bevorzugter mehr als 90°, noch bevorzugter mehr als 100° und am meisten bevorzugt mehr als 105° auf. Ein Wasser-Fortschreit-Kontaktwinkel (θ_{adv}) eines hydrophoben Bereichs, vorzugsweise der zweiten Bereiche, beträgt vorzugsweise mindestens 100°, bevorzugt mehr als 105°, noch bevorzugter mehr als 110°, noch bevorzugter mehr als 115° und am meisten bevorzugt mehr als 120°.

Vorzugsweise haben hydrophile Bereiche, vorzugsweise die ersten Bereiche, einen statischen Wasser-Kontaktwinkel von weniger als 90°, bevorzugt weniger als 70°, noch bevorzugter weniger als 60°, noch bevorzugter weniger als 50°, noch bevorzugter weniger als 40° und am meisten bevorzugt weniger als 30°. In einer bevorzugten Ausführungsform der vorliegenden Erfindung weisen hydrophile Bereiche, vorzugsweise die ersten Bereiche, einen Wasser-Rückzugs-Kontaktwinkel (θ_{rec}) von höchstens 60°, bevorzugt weniger als 30°, noch bevorzugter weniger als 20°, noch bevorzugter weniger als 15° und am meisten bevorzugt weniger als 10° auf. Ein Wasser-Fortschreit-Kontaktwinkel (θ_{adv}) eines hydrophilen Bereichs, vorzugsweise der ersten Bereiche, beträgt vorzugsweise höchstens 80°, bevorzugt weniger als 65°, noch bevorzugter weniger als 50°, noch bevorzugter weniger als 45° und am meisten bevorzugt weniger als 40°.

Im Allgemeinen können Oberflächen in Abhängigkeit ihres Kontaktwinkels mit wässrigen und organischen Flüssigkeiten als omniphil oder omniphob eingestuft werden. Eine Oberfläche mit einem statischen Kontaktwinkel von mindestens 90° wird hierbei als omniphob bezeichnet, während eine Oberfläche mit einem statischen Kontaktwinkel von weniger als 90° als omniphil bezeichnet wird. Die Begriffe "omniphil" und "omniphob" beziehen sich auf wässrige und organische Lösungen.

Sofern nicht anderweitig angegeben, gelten die folgenden Definitionen in Bezug auf die Begriffe "Halogen", "Alkylgruppe", "Cycloalkylgruppe", "Arylgruppe" und "Heteroarylgruppe". Hier bezeichnet der Begriff "Halogen" insbesondere Fluoratome, Chloratome und Bromatome. Ferner bezeichnet der Begriff "Alkylgruppe" eine verzweigte oder unverzweigte Alkylgruppe mit 1 bis 20, vorzugsweise 1 bis 12, noch bevorzugter 1 bis 6 Kohlenstoffatomen, wobei die Alkylgruppe substituiert oder unsubstituiert sein kann. Der Begriff "Cycloalkylgruppe" bezeichnet eine Cycloalkylgruppe mit 3 bis 10, vorzugsweise 4 bis 8, noch bevorzugter 5 oder 6 Kohlenstoffatomen, wobei die Cycloalkylgruppe substituiert oder unsubstituiert sein kann. Der Begriff "Arylgruppe" bezeichnet eine Arylgruppe, bestehend aus beispielsweise 1 bis 3 aromatischen Ringen, wie beispielsweise eine Phenylgruppe oder eine Naphthylgruppe, wobei die Arylgruppe substituiert oder unsubstituiert sein kann. Der Begriff "Heteroarylgruppe" bezeichnet eine Heteroarylgruppe, bestehend aus beispielsweise 1 bis 3 aromatischen Ringen mit Heteroatomen, wie beispielsweise eine Pyridylgruppe, eine Pyrimidinylgruppe, eine Thienylgruppe, eine Furylgruppe oder eine Pyrrolylgruppe, wobei die Heteroarylgruppe substituiert oder unsubstituiert sein kann.

Wie vorstehend ausgeführt können die Alkylgruppe, die Cycloalkylgruppe, die Arylgruppe und die Heteroarylgruppe substituiert oder unsubstituiert sein. Ferner können in den hier angegebenen allgemeinen Formeln, sofern nicht anderweitig angegeben, die Kettenkohlenstoffatome substituiert oder unsubstituiert sein. Die möglichen Substituenten unterliegen keiner besonderen Beschränkung, d.h. anstelle von Wasserstoffatomen können sämtliche im Stand der Technik bekannten Substituenten an den weiteren Positionen der entsprechenden Gruppe gebunden sein. Beispielsweise sind die möglichen Substituenten ausgewählt aus der Gruppe, bestehend aus einer verzweigten oder unverzweigten Alkylgruppe mit 1 bis 6 Kohlenstoffatomen, einer Cycloalkylgruppe mit 4 bis 8 Kohlenstoffatomen, einer Arylgruppe mit 1 bis 3 aromatischen Ringen, einer Heteroarylgruppe mit 1 bis 3 aromatischen Ringen mit Heteroatomen, -NL¹L², -NO₂, -CN, -OL³, -C(O)L⁴, -C(O)NL⁵L⁶, -COOL⁷ und -SO₃L⁸, wobei L¹ bis L⁸ unabhängig voneinander ausgewählt sind aus der Gruppe, bestehend aus Wasserstoff, einer verzweigten oder unverzweigten Alkylgruppe mit 1 bis 6 Kohlenstoffatomen, einer Cycloalkylgruppe mit 4 bis 8 Kohlenstoffatomen, einer Arylgruppe mit 1 bis 3 aromatischen Ringen, einer Heteroarylgruppe mit 1 bis 3 aromatischen Ringen mit Heteroatomen.

Vorzugsweise sind die möglichen Substituenten ausgewählt aus der Gruppe, bestehend aus einer verzweigten oder unverzweigten Alkylgruppe mit 1 bis 6 Kohlenstoffatomen, einer Arylgruppe mit 1 bis 3 aromatischen Ringen, einer Heteroarylgruppe mit 1 bis 3 aromatischen Ringen mit Heteroatomen, -NH₂, -NHCH₃, -N(CH₃)₂, -NO₂, -OH, -OCH₃, -OEt, -C(O)H und -COOH. Ferner können ein oder mehrere tetravalente Kohlenstoffatome (zusammen mit den daran gebundenen Wasserstoffatomen), falls vorhanden, in den Alkylgruppen und Cycloalkylgruppen unabhängig voneinander substituiert sein mit einem Mitglied, ausgewählt aus der Gruppe, bestehend aus O, (OCH₂CH₂)ₐO, S, (SCH₂CH₂)_{b}S, C(O), C(O)O, NL⁹ und C(O)NL¹⁰, vorzugsweise bestehend aus O, (OCH₂CH₂)ₐO, C(O)O und C(O)NL¹⁰, wobei a und b unabhängig voneinander eine ganze Zahl von 1 bis 6 sind. L⁹ und L¹⁰ sind unabhängig voneinander ausgewählt aus der Gruppe, bestehend aus Wasserstoff, einer verzweigten oder unverzweigten Alkylgruppe mit 1 bis 6 Kohlenstoffatomen, einer Cycloalkylgruppe mit 4 bis 8 Kohlenstoffatomen, einer Arylgruppe mit 1 bis 3 aromatischen Ringen, einer Heteroarylgruppe mit 1 bis 3 aromatischen Ringen mit Heteroatomen.

Vorzugsweise sind die Alkylgruppe, die Cycloalkylgruppe, die Arylgruppe und die Heteroarylgruppe unsubstituiert. Die Alkylgruppe ist vorzugsweise unverzweigt. Vorzugsweise sind in den hier angegebenen allgemeinen Formeln die Kettenkohlenstoffatome substituiert oder unsubstituiert sein.

In einer bevorzugten Ausführungsform der vorliegenden Erfindung weisen die (ersten und zweiten) Dendrimerstrukturen jeweils eine Alkylen-Silyl-Gruppe auf und sind die Dendrimerstrukturen mittels der jeweiligen Alkylen-Silyl-Gruppe mit der Substratoberfläche (über die Sauerstoffatome der Hydroxylgruppen oder Silanolgruppen der Substratoberfläche) verbunden. Die Alkylen-Silyl-Gruppen können als Kern der entsprechenden bevorzugten Dendrimerstrukturen angesehen werden. Vorzugsweise binden die Siliziumatome an die Hydroxylgruppen oder Silanolgruppen der Substratoberfläche über-Si-O-Bindungen und die Alkylen-Reste an die repetitiven Einheiten, wie in Figur 1 beispielhaft gezeigt (Siliziumatom nicht gezeigt). Die Alkylen-Silyl-Gruppen der ersten und zweiten Dendrimerstrukturen können gleich oder verschieden sein. Vorzugsweise sind die Alkylen-Silyl-Gruppen gleich, da dadurch der Synthesesaufwand gering gehalten werden kann.

Vorzugsweise weist die Alkylen-Silyl-Gruppe (der ersten und/oder zweiten Dendrimerstrukturen) eine Struktur der folgenden allgemeinen Formel (1) auf, die substituiert oder unsubstituiert sein kann, 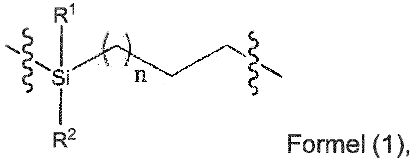 wobei R¹ und R² unabhängig voneinander ausgewählt sind aus der Gruppe, bestehend aus einer Alkylgruppe, einer Cycloalkylgruppe, einer Arylgruppe, einer Heteroarylgruppe, -OA, wobei A unabhängig voneinander ausgewählt ist aus der Gruppe, bestehend aus einer Alkylgruppe, einer Cycloalkylgruppe, einer Arylgruppe und einer Heteroarylgruppe; und n eine ganze Zahl von 0 bis 6 ist. Vorzugsweise ist A eine Alkylgruppe oder eine Arylgruppe, noch bevorzugter ist A eine Alkylgruppe, beispielswiese eine Alkylgruppe mit 1 bis 6 Kohlenstoffatomen, vorzugsweise mit 1 oder 2 Kohlenstoffatomen. Noch bevorzugter ist A eine Ethylgruppe. Vorzugsweise sind R¹ und R² unabhängig voneinander eine Alkylgruppe, eine Arylgruppe oder -OA, noch bevorzugter eine Alkylgruppe oder -OA und noch bevorzugter -OA. R¹ und R² sind beispielsweise jeweils eine Methylgruppe, eine Methoxygruppe oder eine Ethoxygruppe, vorzugsweise jeweils eine Methoxygruppe oder eine Ethoxygruppe, noch bevorzugter jeweils eine Ethoxygruppe. Vorzugsweise ist n eine ganze Zahl von 0 bis 4, noch bevorzugter von 0 bis 2 und noch bevorzugter von 0 oder 1 und am meisten bevorzugt ist n 0. Die Alkylen-Silyl-Gruppe weist vorzugsweise eine Struktur der folgenden allgemeinen Formel (1a) oder (1b), am meisten bevorzugt eine Struktur der folgenden allgemeinen Formel (1b), auf.

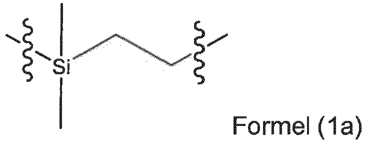

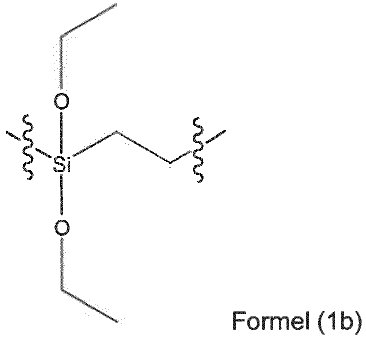

In einer bevorzugten Ausführungsform der vorliegenden Erfindung weisen die ersten Dendrimerstrukturen und die zweiten Dendrimerstrukturen jeweils repetitive Einheiten mit der folgenden allgemeinen Formel (2) oder (3), die substituiert oder unsubstituiert sein können, vorzugsweise jeweils repetitive Einheiten mit der folgenden allgemeinen Formel (2), auf 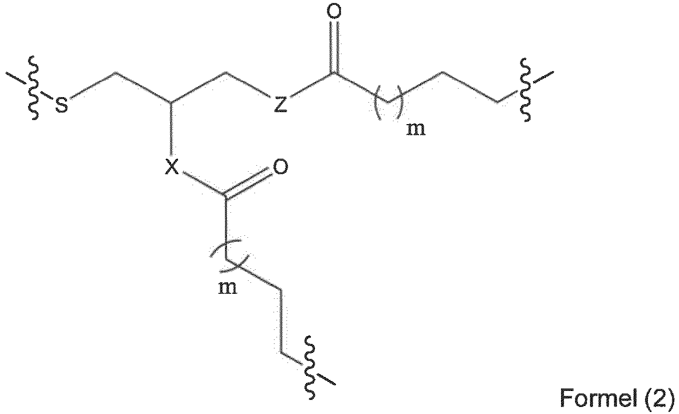 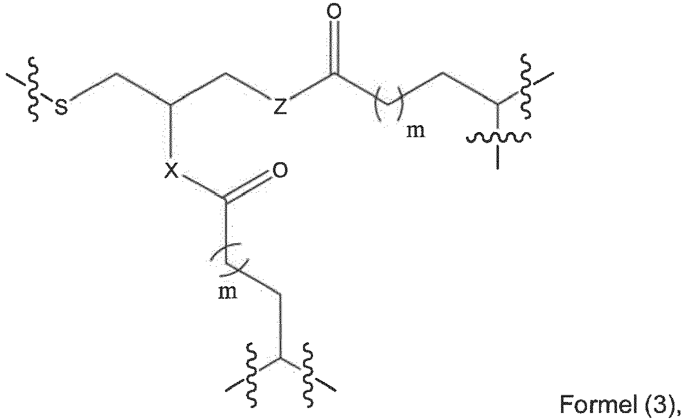 wobei X und Z unabhängig voneinander NR³ oder O sind, wobei jedes R³ unabhängig voneinander ausgewählt ist aus der Gruppe, bestehend aus Wasserstoff, einer Alkylgruppe, einer Cycloalkylgruppe, einer Arylgruppe und einer Heteroarylgruppe; und m eine ganze Zahl von mindestens 1 ist. Vorzugsweise ist R³ Wasserstoff oder eine Alkylgruppe, noch bevorzugter Wasserstoff. In einer bevorzugten Ausführungsform sind X und Z jeweils O. Vorzugsweise ist m eine ganze Zahl von 1 bis 6, noch bevorzugter von 1 bis 4, noch bevorzugter von 1 oder 2 und am meisten bevorzugt ist m 1. Die ersten Dendrimerstrukturen und die zweiten Dendrimerstrukturen weisen vorzugweise jeweils repetitive Einheiten mit der folgenden allgemeinen Formel (2a) oder (3a), noch bevorzugter jeweils repetitive Einheiten mit der folgenden allgemeinen Formel (2a), auf.

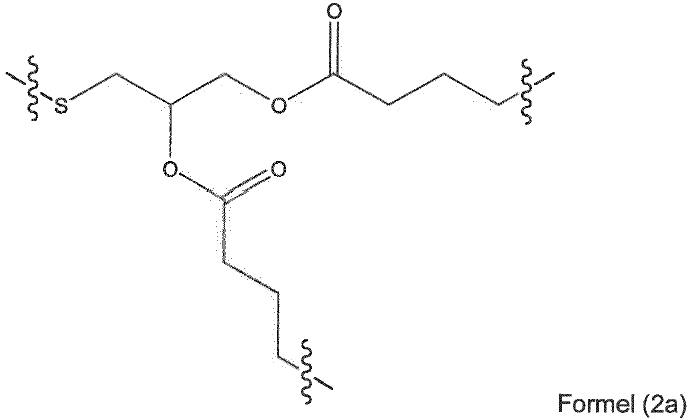

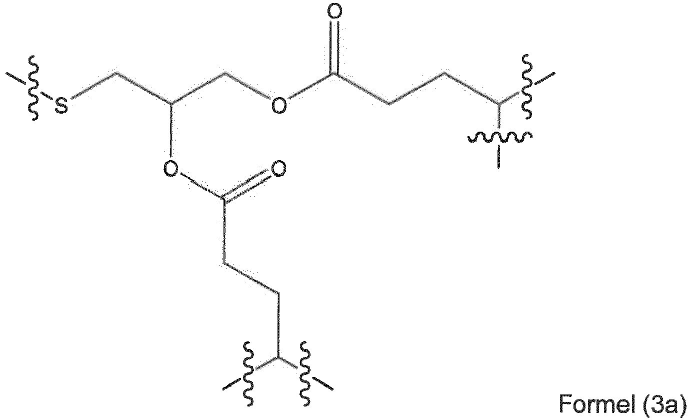

Die ersten Dendrimerstrukturen weisen vorzugweise Endgruppen mit der folgenden allgemeinen Formel (4) oder (5), die substituiert oder unsubstituiert sein können, vorzugsweise Endgruppen mit der folgenden allgemeinen Formel (4), auf.

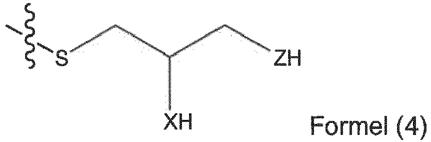

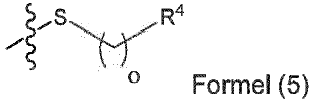

Für X und Z in der allgemeinen Formel (4) gelten die vorstehenden Definitionen und Ausführungsformen bzgl. X und Z der repetitiven Einheiten mit der allgemeinen Formel (2) oder (3) analog. Vorzugsweise weisen die Endgruppen mit der allgemeinen Formel (4) die folgende Formel (4a) auf.

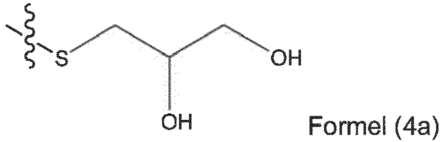

In der allgemeinen Formel (5) ist R⁴ ausgewählt aus der Gruppe, bestehend aus -NG¹G², -NO₂, -CN, -OG³, -C(O)G⁴, -C(O)NG⁵G⁶, -COOG⁷ und -SO₃G⁸, wobei G¹ bis G⁸ unabhängig voneinander ausgewählt sind aus der Gruppe, bestehend aus Wasserstoff, einer Alkylgruppe, einer Cycloalkylgruppe, einer Arylgruppe und einer Heteroarylgruppe. Vorzugsweise sind G¹ bis G⁸ unabhängig voneinander Wasserstoff oder eine Alkylgruppe, noch bevorzugter Wasserstoff. In einer bevorzugten Ausführungsform ist R⁴ -NG¹G² oder -OG³, noch bevorzugter-NG¹G², und am meisten bevorzugt -NH₂. Ferner ist o eine ganze Zahl von 1 bis 6 in der allgemeinen Formel (5). Vorzugsweise ist o eine ganze Zahl von 1 bis 4 und noch bevorzugter von 2 oder 3 und am meisten bevorzugt ist o 2. In einer bevorzugten Ausführungsform weisen die Endgruppen mit der allgemeinen Formel (5) die folgende Formel (5a) auf.

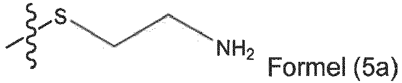

Die ersten Dendrimerstrukturen weisen am meisten bevorzugt Endgruppen mit der allgemeinen Formel (4a) auf.

Die zweiten Dendrimerstrukturen weisen vorzugsweise Endgruppen mit der folgenden allgemeinen Formel (6), die substituiert oder unsubstituiert sein können, auf 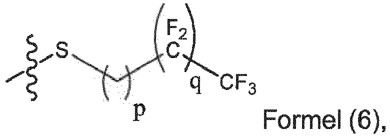 wobei p eine ganze Zahl von 0 bis 10 ist und q eine ganze Zahl von 3 bis 15 ist. Vorzugsweise ist p eine ganze Zahl von 0 bis 6 und noch bevorzugter von 1 bis 3 und am meisten bevorzugt ist p 2. Ferner ist q vorzugweise eine ganze Zahl von 4 bis 12, noch bevorzugter von 5 bis 9 und noch bevorzugter von 6 bis 8 und am meisten bevorzugt ist q 7. Am meisten bevorzugt weisen die zweiten Dendrimerstrukturen Endgruppen mit der folgenden allgemeinen Formel (6a) auf.

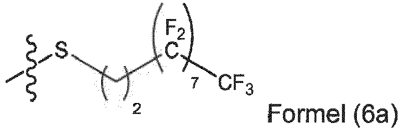

In einer besonders bevorzugten Ausführungsform der vorliegenden Erfindung weisen die ersten und zweiten Dendrimerstrukturen jeweils eine Alkylen-Silyl-Gruppe mit einer Struktur der allgemeinen Formel (1b) und jeweils drei (aufeinanderfolgende) repetitive Einheiten mit der allgemeinen Formel (2a) auf, weisen die ersten Dendrimerstrukturen Endgruppen mit der allgemeinen Formel (4a) auf und weisen die zweiten Dendrimerstrukturen Endgruppen mit der allgemeinen Formel (6a) auf. Entsprechende Dendrimerstrukturen (vor Einfügung der Endgruppen) sind in Figur 1 beispielhaft gezeigt (Siliziumatom der Alkylen-Silyl-Gruppe nicht gezeigt).

Ein weiterer Aspekt der vorliegenden Erfindung betrifft insbesondere ein Verfahren zur Herstellung eines erfindungsgemäßen gemusterten Substrats, umfassend das Aufbringen zweiter Dendrimerstrukturen in zweiten Bereichen und erster Dendrimerstrukturen in ersten Bereichen auf einer Oberfläche des Substrats, wobei die Dendrimerstrukturen jeweils kovalent mit der Substratoberfläche verbunden sind; die zweiten Bereiche die ersten Bereiche umschließen; und die zweiten Dendrimerstrukturen mindestens ein Strukturelement aufweisen, das sich von den Strukturelementen der ersten Dendrimerstrukturen unterscheidet. Die vorstehenden Definitionen und Ausführungsformen gelten analog für diesen Aspekt der vorliegenden Erfindung.

Vorzugsweise umfasst das erfindungsgemäße Herstellungsverfahren die Schritte
(a) Bereitstellen eines Substrats, das eine Oberfläche umfasst, die Hydroxylgruppen oder Silanolgruppen aufweist;
(b) Umsetzen der Hydroxylgruppen oder Silanolgruppen dieser Oberfläche mit einem Alkenyl-Silan der folgenden allgemeinen Formel (12), um Alkenyl-Silyl-Gruppen auf der Oberfläche zu bilden 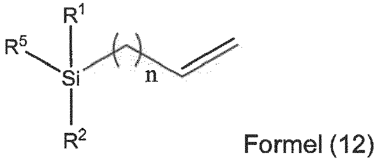 wobei R¹ und R² unabhängig voneinander ausgewählt sind aus der Gruppe, bestehend aus einer Alkylgruppe, einer Cycloalkylgruppe, einer Arylgruppe, einer Heteroarylgruppe, -OA, wobei A unabhängig voneinander ausgewählt ist aus der Gruppe, bestehend aus einer Alkylgruppe, einer Cycloalkylgruppe, einer Arylgruppe und einer Heteroarylgruppe;
   R⁵ ein Halogen oder -OQ ist, wobei Q ausgewählt ist aus der Gruppe, bestehend aus einer Alkylgruppe, einer Cycloalkylgruppe, einer Arylgruppe und einer Heteroarylgruppe; und
   n eine ganze Zahl von 0 bis 6 ist;
(c) Umsetzen mit einer Thiolverbindung der folgenden allgemeinen Formel (7) 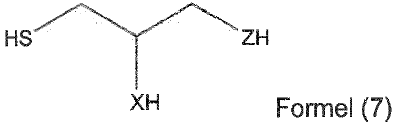 wobei X und Z unabhängig voneinander NR³ oder O sind, wobei jedes R³ unabhängig voneinander ausgewählt ist aus der Gruppe, bestehend aus Wasserstoff, einer Alkylgruppe, einer Cycloalkylgruppe, einer Arylgruppe und einer Heteroarylgruppe;
(d) Umsetzen mit einer Carbonsäure der folgenden allgemeinen Formel (8) oder (9) 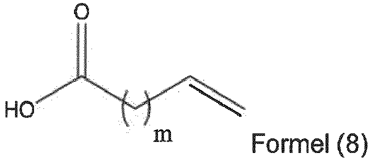 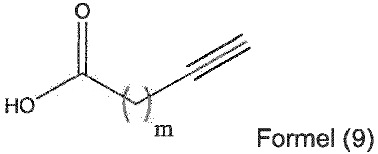 wobei m eine ganze Zahl von mindestens 1 ist;
(e) Mindestens 1-maliges Wiederholen der Schritte (c) und (d) in Kombination;
(f) Gezieltes Umsetzen eines Teiles der Alkenyl- oder Alkinyl-Gruppen, erhalten aus dem letztmaligen Ausführen des Schritts (d), entweder mit
   (i) einer Thiolverbindung der folgenden allgemeinen Formel (10), um zweite Dendrimerstrukturen zu erhalten 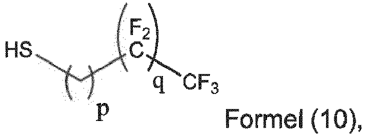
      wobei p eine ganze Zahl von 0 bis 10 ist und
      q eine ganze Zahl von 3 bis 15 ist; oder mit
   (ii) einer Thiolverbindung der allgemeinen Formel (7) oder einer Thiolverbindung der folgenden allgemeinen Formel (11), um erste Dendrimerstrukturen zu erhalten 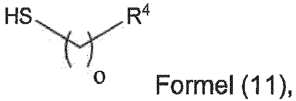 wobei R⁴ ausgewählt ist aus der Gruppe, bestehend aus -NG¹G², -NO₂, -CN, -OG³, -C(O)G⁴, -C(O)NG⁵G⁶, -COOG⁷ und -SO₃G⁸, wobei G¹ bis G⁸ unabhängig voneinander ausgewählt sind aus der Gruppe, bestehend aus Wasserstoff, einer Alkylgruppe, einer Cycloalkylgruppe, einer Arylgruppe und einer Heteroarylgruppe; und
      o eine ganze Zahl von 1 bis 6 ist;
(g) Umsetzen der übrigen Alkenyl- oder Alkinyl-Gruppen entweder mit
   (i) einer Thiolverbindung der allgemeinen Formel (7) oder einer Thiolverbindung der allgemeinen Formel (11), um erste Dendrimerstrukturen zu erhalten, falls in Schritt (f) zweite Dendrimerstrukturen (gemäß (i) in Schritt (f)) gebildet wurden; oder mit
   (ii) einer Thiolverbindung der allgemeinen Formel (10), um zweite Dendrimerstrukturen zu erhalten, falls in Schritt (f) erste Dendrimerstrukturen (gemäß (ii) in Schritt (f)) gebildet wurden.

In einer bevorzugten Ausführungsform umfasst der Schritt (a) den Schritt (a1) des Aktivierens der Oberfläche des Substrats. Die Mittel zur Aktivierung unterliegen keinen besonderen Einschränkungen. Beispielsweise kann die Substratoberfläche mittels Plasmabehandlung oder aufeinanderfolgender Behandlung mit NaOH und HCl, vorzugsweise mittels Plasmabehandlung, aktiviert werden. Die Plasmabehandlung kann beispielsweise für 1 min bis 2 h, vorzugsweise für 2 min bis 40 min und am meisten bevorzugt für 5 min bis 15 min, bei beispielsweise von 0 bis 80°C, vorzugsweise von 15 bis 40°C, und am meisten bevorzugt von 20 bis 30°C, durchgeführt werden.

Im Schritt (b) werden die Hydroxylgruppen oder Silanolgruppen der Oberfläche des Substrats mit einem Alkenyl-Silan der allgemeinen Formel (12), das substituiert oder unsubstituiert sein kann, umgesetzt, um Alkenyl-Silyl-Gruppen auf der Oberfläche zu bilden. Über diese Alkylen-Silyl-Gruppen können die ersten und zweiten Dendrimerstrukturen mit der Substratoberflächen verbunden sein. Für R¹, R² und n in der allgemeinen Formel (12) gelten die vorstehenden Definitionen und Ausführungsformen bzgl. R¹, R² und n der Struktur der allgemeinen Formel (1) analog. R⁵ ist ein Halogen oder -OQ, wobei Q ausgewählt ist aus der Gruppe, bestehend aus einer Alkylgruppe, einer Cycloalkylgruppe, einer Arylgruppe und einer Heteroarylgruppe. Das Halogen der Gruppe R⁵ kann beispielsweise Chlor, Brom, oder lod sein. Vorzugsweise ist das Halogen Chlor. Vorzugsweise ist Q eine Alkylgruppe oder eine Arylgruppe, noch bevorzugter ist Q eine Alkylgruppe, beispielswiese eine Alkylgruppe mit 1 bis 6 Kohlenstoffatomen, vorzugsweise mit 1 oder 2 Kohlenstoffatomen. Noch bevorzugter ist Q eine Ethylgruppe. R⁵ ist vorzugsweise -OQ, noch bevorzugter ist -OQ eine Ethoxygruppe. Das Alkenyl-Silan ist vorzugsweise Chlor(dimethyl)vinylsilan oder Triethoxyvinylsilan, am meisten bevorzugt Triethoxyvinylsilan.

Die (Reaktions-)Temperatur im Schritt (b) unterliegt keinen besonderen Beschränkungen. Es können somit übliche Temperaturen vom Fachmann verwendet werden. In einer bevorzugten Ausführungsform ist die (Reaktions-)Temperatur im Schritt (b) von 10 bis 150°C, noch bevorzugter von 30 bis 120°C, noch bevorzugter von 50 bis 100°C, noch bevorzugter von 70 bis 90°C und am meisten bevorzugt 80°C.

Die (Reaktions-)Zeit im Schritt (b) unterliegt keinen besonderen Beschränkungen. Es können somit übliche Dauern vom Fachmann angesetzt werden. In einer bevorzugten Ausführungsform ist die (Reaktions-)Zeit im Schritt (b) von 30 min bis 48 h, noch bevorzugter von 2 h bis 36 h, noch bevorzugter von 6 h bis 24 h und am meisten bevorzugt von 10 h bis 14 h.

Die Umsetzung in Schritt (b) kann durch Aufbringen einer Lösung des Alkenyl-Silans auf die Oberfläche des Substrats oder durch Umsetzung in der Gasphase erfolgen. Vorzugsweise erfolgt die Umsetzung in der Gasphase. Die Umsetzung in der Gasphase erfolgt vorzugsweise unter reduziertem Druck. Dabei kann beispielsweise ein Druck von 1 mbar bis 800 mbar, vorzugsweise von 10 mbar bis 400 mbar, noch bevorzugter von 20 mbar bis 200 mbar und am meisten bevorzugt von 30 mbar bis 100 mbar angewandt werden.

In den Schritten (c) und (d) werden die repetitiven Einheiten der Dendrimerstrukturen aufgebaut. Pro Durchführung der Kombination aus den Schritten (c) und (d) wird eine Generation von repetitiven Einheiten erhalten.

In Schritt (c) erfolgt das Umsetzen mit einer Thiolverbindung der allgemeinen Formel (7), die substituiert oder unsubstituiert sein kann. Für X und Z in der allgemeinen Formel (7) gelten die vorstehenden Definitionen und Ausführungsformen bzgl. X und Z der repetitiven Einheiten mit der allgemeinen Formel (2) oder (3) analog. Vorzugsweise ist die Thiolverbindung der allgemeinen Formel (7) Thioglycerol mit der folgenden Formel (7a).

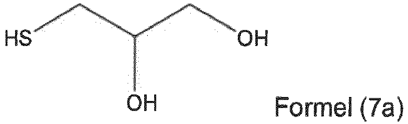

Beim ersten Durchführen des Schritts (c) werden die Alkenyl-Gruppen des Alkenyl-Silans aus Schritt (b) mit der Thiolverbindung der allgemeinen Formel (7) umgesetzt. Bei den weiteren Durchführungen des Schritts (c) werden die Alkenyl- oder Alkinyl-Gruppen der Carbonsäuren aus den davor durchgeführten Schritt (d) umgesetzt. Entsprechende Umsetzungen sind aus dem Stand der Technik bekannt. Vorzugsweise erfolgt die Umsetzung mittels Thiol-En-Click- bzw. Thiol-In-Click-Umsetzungen.

Die Umsetzung in Schritt (c) unterliegt keinen besonderen Beschränkungen. Beispielsweise kann es sich um Thiol-En-Click- bzw. Thiol-In-Click-Umsetzungen handeln. In einer bevorzugten Ausführungsform erfolgt die Umsetzung in Schritt (c) durch eine Photoreaktion, d.h. durch Licht stimuliert, vorzugsweise mit UV-Licht. Die Lichtquelle unterliegt keinen besonderen Beschränkungen. Es kann beispielsweise mit Licht einer Wellenlänge von 200 bis 360 nm, vorzugsweise 260 nm, bei einer Intensität von 0,5 bis 20 mW cm⁻², vorzugsweise von 2 bis 5 mW cm⁻², bestrahlt werden. Das Licht, das von der Lichtquelle emittiert wird, kann die Reaktion initiieren oder kann dazu verwendet werden, einen optionalen Photoinitiator anzuregen, der in dem Wellenlängenbereich des Lichtes, das von der Lichtquelle emittiert wird, einen Absorptionsbereich aufweist.

In einer bevorzugten Ausführungsform des erfindungsgemäßen Herstellungsverfahrens Schritt (c) in Gegenwart eines Photoinitiators, wie beispielsweise 2,2-Dimethoxy-2-phenylacetophenon (DMPAP), der nach Lichtabsorption in reaktive Spezies zerfällt, die weitere Reaktionen initiieren. Der Photoinitiator kann beispielsweise in einer Konzentration von 1,0 bis 30 mg mL⁻¹, insbesondere von 1,0 bis 20 mg mL⁻¹, und am meisten bevorzugt von 5,0 bis 15 mg mL⁻¹ neben der Thiolverbindung der allgemeinen Formel (7) vorliegen. Der Photoinitiator kann gemeinsam mit der Thiolverbindung der allgemeinen Formel (7) aufgebracht werden.

Die (Reaktions-)Temperatur im Schritt (c) unterliegt keinen besonderen Beschränkungen. Es können somit übliche Temperaturen vom Fachmann verwendet werden. In einer bevorzugten Ausführungsform ist die (Reaktions-)Temperatur im Schritt (c) von 0 bis 80°C, noch bevorzugter von 10 bis 60°C, noch bevorzugter von 15 bis 40°C, und am meisten bevorzugt von 20 bis 30°C.

Die (Reaktions-)Zeit im Schritt (c) unterliegt keinen besonderen Beschränkungen. Es können somit übliche Dauern vom Fachmann angesetzt werden. In einer bevorzugten Ausführungsform ist die (Reaktions-)Zeit im Schritt (c) von 10 sec bis 40 min, noch bevorzugter von 30 sec bis 15 min, und am meisten bevorzugt von 1 min bis 3 min.

Die Umsetzung in Schritt (c) kann durch Aufbringen der Thiolverbindung der allgemeinen Formel (7) oder einer Lösung davon auf die Oberfläche des Substrats erfolgen. Vorzugsweise erfolgt die Umsetzung mit einer Lösung der Thiolverbindung der allgemeinen Formel (7), beispielsweise einer 1 bis 40 vol%-igen Lösung, vorzugsweise einer 5 bis 20 vol%-igen Lösung und am meisten bevorzugt einer 10 vol%-igen Lösung. Das Reaktionsmedium (Medium) für Schritt (c) unterliegt dabei keinen besonderen Beschränkungen. Beispielsweise kann es sich bei dem Medium um ein wässriges Medium, ein Medium auf Basis eines oder mehrerer organischer Lösungsmittel oder um ein Medium auf Basis eines Gemisches aus Wasser und einem oder mehreren organischen Lösungsmitteln handeln. Vorzugsweise wird das Reaktionsmedium für Schritt (c) des erfindungsgemäßen Herstellungsverfahrens ausgewählt aus der Gruppe, bestehend aus Wasser, Ethanol, Methanol, Isopropanol und Aceton. Am bevorzugtesten ist das Reaktionsmedium für Schritt (c) des erfindungsgemäßen Herstellungsverfahrens ein Gemisch aus Wasser und Ethanol, vorzugsweise im Volumenverhältnis 1:1.

In Schritt (d) erfolgt das Umsetzen mit einer Carbonsäure der allgemeinen Formel (8) oder (9), die substituiert oder unsubstituiert sein kann, vorzugsweise mit einer Carbonsäure der allgemeinen Formel (8). Für m in den allgemeinen Formeln (8) und (9) gelten die vorstehenden Definitionen und Ausführungsformen bzgl. m der repetitiven Einheiten mit der allgemeinen Formel (2) oder (3) analog. Vorzugsweise ist die Carbonsäure der allgemeinen Formel (8) 4-Pentensäure und die Carbonsäure der allgemeinen Formel (9) 4-Pentinsäure. Im Schritt (d) werden die funktionellen Gruppen XH und ZH, die durch die Umsetzung mit der Thiolverbindung der allgemeinen Formel (7) im Schritt (c) vorhanden sind mit der Carbonsäure in Schritt (d) umgesetzt, wodurch (zusammen mit Schritt (c)) eine (weitere) Generation der repetitiven Einheit erhalten wird.

Die Umsetzung in Schritt (d) unterliegt keinen besonderen Beschränkungen. Beispielsweise kann das Substrat aus Schritt (c) mit einer Lösung, die die Carbonsäure der allgemeinen Formel (8) oder (9) enthält, behandelt werden, bspw. durch Eintauchen des Substrats in die Lösung.

In einer bevorzugten Ausführungsform des erfindungsgemäßen Herstellungsverfahrens erfolgt Schritt (d) in Gegenwart eines nukleophilen Katalysators, wie beispielsweise 4-(Dimethylamino)pyridin (DMAP). Der nukleophile Katalysator kann beispielsweise in einer Konzentration von 0,1 bis 50 mg mL⁻¹, insbesondere von 1,0 bis 10 mg mL⁻¹, und am meisten bevorzugt von 1,0 bis 5,0 mg mL⁻¹ neben der Carbonsäure vorliegen.

Vorzugsweise erfolgt Schritt (d) in Gegenwart eines Kupplungsreagenzes, wie beispielsweise N,N'-Diisopropylcarbodiimid (DIC) oder Dicyclohexylcarbodiimid (DCC), vorzugsweise N,N'-Diisopropylcarbodiimid (DIC). Das Kupplungsreagenz kann beispielsweise in einer Konzentration von 0,1 bis 1,0 Vol%, insbesondere von 0,1 bis 0,5 Vol%, und am meisten bevorzugt von 0,1 bis 0,3 Vol% neben der Carbonsäure vorliegen.

Die (Reaktions-)Temperatur im Schritt (d) unterliegt keinen besonderen Beschränkungen. Es können somit übliche Temperaturen vom Fachmann verwendet werden. In einer bevorzugten Ausführungsform ist die (Reaktions-)Temperatur im Schritt (d) von -40 bis 60°C, noch bevorzugter von -20 bis 50°C, noch bevorzugter von 10 bis 40°C, und am meisten bevorzugt von 20 bis 30°C.

Die (Reaktions-)Zeit im Schritt (d) unterliegt keinen besonderen Beschränkungen. Es können somit übliche Dauern vom Fachmann angesetzt werden. In einer bevorzugten Ausführungsform ist die (Reaktions-)Zeit im Schritt (d) von 30 min bis 24 h, noch bevorzugter von 1 h bis 12 h, noch bevorzugter von 2 h bis 8 h und am meisten bevorzugt von 3 h bis 5 h.

Vorzugsweise erfolgt die Umsetzung in Schritt (d) mit einer Lösung der Carbonsäure der allgemeinen Formel (8) oder (9). Die Carbonsäure der allgemeinen Formel (8) oder (9) kann beispielsweise in einer Konzentration von 1,0 bis 50 mg mL⁻¹, insbesondere von 1,0 bis 20 mg mL⁻¹, und am meisten bevorzugt von 1,0 bis 5,0 mg mL⁻¹ vorliegen. Das Reaktionsmedium (Medium) im Schritt (d) unterliegt dabei keinen besonderen Beschränkungen. Beispielsweise kann es sich bei dem Medium um ein wässriges Medium, ein Medium auf Basis eines oder mehrerer organischer Lösungsmittel oder um ein Medium auf Basis eines Gemisches aus Wasser und einem oder mehreren organischen Lösungsmitteln handeln. Vorzugsweise wird das Reaktionsmedium für Schritt (d) des erfindungsgemäßen Herstellungsverfahrens ausgewählt aus der Gruppe, bestehend aus Aceton und Dichlormethan (DCM). Am bevorzugtesten ist das Reaktionsmedium für Schritt (d) des erfindungsgemäßen Herstellungsverfahrens Aceton.

In Schritt (e) erfolgt das mindestens 1-malige Wiederholen der Schritte (c) und (d) in Kombination, d.h. die Schritte (c) und (d) werden im erfindungsgemäßen Herstellungsverfahren mindestens zwei Mal hintereinander (d.h. c, d, c, d) durchgeführt. Dadurch können mindestens zwei Generationen an repetitiven Einheiten in den Dendrimerstrukturen gebildet werden. Die maximale Anzahl an Wiederholungen unterliegt keinen besonderen Beschränkungen. Vorzugsweise wird die Kombination aus den Schritten (c) und (d) 1 bis 5 Mal, noch bevorzugter 1 bis 3 Mal und am meisten bevorzugt 2 Mal (d.h. Bildung von drei Generationen an repetitiven Einheiten in den Dendrimerstrukturen), wiederholt.

Durch diese dendrimere Oberflächenmodifizierung kann die Reaktivität der Oberfläche mit jedem Durchlauf des Zyklus gesteigert werden. Nach jedem Durchlauf verdoppelt sich die Anzahl der reaktiven Doppelbindungen auf der Oberfläche bspw. bei Verwendung der Thiolverbindung der allgemeinen Formel (7) und der Carbonsäure der allgemeinen Formel (8). Die Dichte der funktionellen Gruppen auf gleichem Raum nimmt stetig zu. Nach dem dritten Durchlauf des Zyklus kann bspw. die Anzahl von ursprünglich einer immobilisierten Doppelbindung auf acht Doppelbindungen erhöht werden, wie in Figur 1 gezeigt.

In Schritt (f) erfolgt das gezielte Umsetzen eines Teiles der Alkenyl- oder Alkinyl-Gruppen, erhalten aus dem letztmaligen Ausführen des Schritts (d). Dabei können zunächst entweder zweite Dendrimerstrukturen (gemäß (i)) mit einer Thiolverbindung der allgemeinen Formel (10), die substituiert oder unsubstituiert sein kann, gebildet werden oder erste Dendrimerstrukturen (gemäß (ii)) mit einer Thiolverbindung der allgemeinen Formel (7) oder einer Thiolverbindung der allgemeinen Formel (11), vorzugsweise mit einer Thiolverbindung der allgemeinen Formel (7), die jeweils substituiert oder unsubstituiert sein können, gebildet werden. Für p und q in der allgemeinen Formel (10) gelten die vorstehenden Definitionen und Ausführungsformen bzgl. p und q der Endgruppen mit der allgemeinen Formel (6) analog. Vorzugsweise ist die Thiolverbindung der allgemeinen Formel (10) 1*H*, 1*H*, 2*H*, 2*H*-Perfluordecanthiol (PFDT). Für X und Z in der allgemeinen Formel (7) gelten die vorstehenden Definitionen und Ausführungsformen in Schritt (c) analog. Für R⁴ und o in der allgemeinen Formel (11) gelten die vorstehenden Definitionen und Ausführungsformen bzgl. R⁴ und o der Endgruppen mit der allgemeinen Formel (5) analog. Vorzugsweise ist die Thiolverbindung der allgemeinen Formel (11) Cysteamin, am bevorzugtesten ist die Thiolverbindung der allgemeinen Formel (7) Thioglycerol. Die entsprechenden Verbindungen können auch als die entsprechenden Salze verwendet werden, bspw. als entsprechende Hydrochloride, wie etwa Cysteamin-Hydrochlorid.

In einer bevorzugten Ausführungsform erfolgt in Schritt (f) das gezielte Umsetzen des Teiles der Alkenyl- oder Alkinyl-Gruppen, erhalten aus dem letztmaligen Ausführen des Schritts (d), mit einer Thiolverbindung der allgemeinen Formel (10), die substituiert oder unsubstituiert sein kann, um zweite Dendrimerstrukturen zu erhalten (gemäß (i)).

Die Umsetzung in Schritt (f) unterliegt keinen besonderen Beschränkungen. Beispielsweise kann es sich um gezielte Thiol-En-Click- bzw. Thiol-In-Click-Umsetzungen unter Verwendung einer Photomaske oder eines Laserstrahls handeln.

In einer bevorzugten Ausführungsform umfasst Schritt (f) die folgenden Schritte (f1) bis (f4):
(f1) Aufbringen der entsprechenden Thiolverbindung auf die Oberfläche mit Alkenyloder Alkinyl-Gruppen;
(f2) Bedecken der Oberfläche mit der Thiolverbindung mit einer Photomaske;
(f3) Bestrahlen der Oberfläche mit der Thiolverbindung und der Photomaske mit UV-Licht; und
(f4) Entfernen der Photomaske,

Schritt (f) bzw. Schritt (f1) kann durch Aufbringen der entsprechenden Thiolverbindung oder einer Lösung davon auf die Oberfläche des Substrats, erhalten aus dem letztmaligen Ausführen des Schritts (d), erfolgen. Vorzugsweise erfolgt die Umsetzung mit einer Lösung der entsprechenden Thiolverbindung, beispielsweise einer 1 bis 40 vol%-igen Lösung, vorzugsweise einer 5 bis 20 vol%-igen Lösung und am meisten bevorzugt einer 10 vol%-igen Lösung. Das Reaktionsmedium (Medium) für Schritt (f) unterliegt dabei keinen besonderen Beschränkungen. Beispielsweise kann es sich bei dem Medium um ein wässriges Medium, ein Medium auf Basis eines oder mehrerer organischer Lösungsmittel oder um ein Medium auf Basis eines Gemisches aus Wasser und einem oder mehreren organischen Lösungsmitteln handeln. Vorzugsweise wird das Reaktionsmedium für die Umsetzung gemäß (i) in Schritt (f) des erfindungsgemäßen Herstellungsverfahrens ausgewählt aus der Gruppe, bestehend aus Aceton und Dichlormethan. Am bevorzugtesten ist das Reaktionsmedium für die Umsetzung gemäß (i) in Schritt (f) des erfindungsgemäßen Herstellungsverfahrens Aceton. Vorzugsweise wird das Reaktionsmedium für die Umsetzung gemäß (ii) in Schritt (f) des erfindungsgemäßen Herstellungsverfahrens ausgewählt aus der Gruppe, bestehend aus Wasser und Ethanol. Am bevorzugtesten ist das Reaktionsmedium für die Umsetzung gemäß (ii) in Schritt (f) des erfindungsgemäßen Herstellungsverfahrens ein Gemisch aus Wasser und Ethanol, vorzugsweise im Volumenverhältnis 1:1.

In einer bevorzugten Ausführungsform des erfindungsgemäßen Herstellungsverfahrens erfolgt Schritt (f) in Gegenwart eines Photoinitiators, wie beispielsweise 2,2-Dimethoxy-2-phenylacetophenon (DMPAP). Der Photoinitiator kann in Schritt (f) beispielsweise in einer Konzentration von 1,0 bis 30 mg mL⁻¹, insbesondere von 1,0 bis 20 mg mL⁻¹, und am meisten bevorzugt von 5,0 bis 15 mg mL⁻¹ neben der entsprechenden Thiolverbindung vorliegen. Der Photoinitiator kann gemeinsam mit der entsprechenden Thiolverbindung aufgebracht werden.

Die Photomaske, die in Schritt (f2) verwendet wird, unterliegt keinen besonderen Beschränkungen. Der Fachmann kann daher alle geeigneten aus dem Stand der Technik bekannten Photomasken verwenden. Entsprechende Photomasken weisen bspw. lichtundurchlässige Bereiche mit einem Durchmesser von vorzugsweise 1 nm bis 1 cm und lichtdurchlässige Bereiche mit einem Durchmesser von vorzugsweise 1 nm bis 1 cm auf. Die Größe und Form der lichtundurchlässigen Bereiche ist nicht weiter beschränkt und kann von einem Fachmann entsprechend der gewünschten Ausmaße der ersten und zweiten Bereiche ausgewählt werden. Die lichtdurchlässigen und lichtundurchlässigen Bereiche sind nach den zu bildenden Dendrimerstrukturen (erste oder zweite) im Schritt (f) angeordnet.

Die Lichtquelle für Schritt (f3) unterliegt keinen besonderen Beschränkungen. Es kann beispielsweise mit Licht einer Wellenlänge von 200 bis 360 nm, vorzugsweise 260 nm, bei einer Intensität von 0,5 bis 20 mW cm⁻², vorzugsweise von 2 bis 5 mW cm⁻², bestrahlt werden.

Die (Reaktions-)Temperatur im Schritt (f) bzw. Schritt (f3) unterliegt keinen besonderen Beschränkungen. Es können somit übliche Temperaturen vom Fachmann verwendet werden. In einer bevorzugten Ausführungsform ist die (Reaktions-)Temperatur im Schritt (f) bzw. Schritt (f3) von 0 bis 80°C, noch bevorzugter von 10 bis 60°C, noch bevorzugter von 15 bis 40°C, und am meisten bevorzugt von 20 bis 30°C.

Die (Reaktions-)Zeit im Schritt (f) bzw. Schritt (f3) unterliegt keinen besonderen Beschränkungen. Es können somit übliche Dauern vom Fachmann angesetzt werden. In einer bevorzugten Ausführungsform ist die (Reaktions-)Zeit im Schritt (f) bzw. Schritt (f3) von 10 sec bis 40 min, noch bevorzugter von 30 sec bis 15 min, und am meisten bevorzugt von 1 min bis 3 min.

In Schritt (g) erfolgt das Umsetzen der übrigen Alkenyl- oder Alkinyl-Gruppen entweder mit
(i) einer Thiolverbindung der allgemeinen Formel (7) oder (11), vorzugsweise mit einer Thiolverbindung der allgemeinen Formel (7), um die ersten Dendrimerstrukturen zu erhalten, falls in Schritt (f) zweite Dendrimerstrukturen (gemäß (i) in Schritt (f)) gebildet wurden; oder mit
(ii) einer Thiolverbindung der allgemeinen Formel (10), um die zweiten Dendrimerstrukturen zu erhalten, falls in Schritt (f) erste Dendrimerstrukturen (gemäß (ii) in Schritt (f)) gebildet wurden. Für die Thiolverbindungen gelten die vorstehenden Definitionen und Ausführungsformen in Schritt (f) analog. Die Umsetzung in Schritt (g) unterliegt keinen besonderen Beschränkungen. Beispielsweise kann es sich um Thiol-En-Click- bzw. Thiol-In-Click-Umsetzungen handeln.

In einer bevorzugten Ausführungsform erfolgt in Schritt (g) das Umsetzen der übrigen Alkenyl- oder Alkinyl-Gruppen mit einer Thiolverbindung der allgemeinen Formel (7) oder (11), vorzugsweise mit einer Thiolverbindung der allgemeinen Formel (7), die substituiert oder unsubstituiert sein kann, um erste Dendrimerstrukturen zu erhalten (gemäß (i)).

In einer bevorzugten Ausführungsform umfasst Schritt (g) die folgenden Schritte (g1) und (g2):
(g1) Aufbringen der entsprechenden Thiolverbindung auf die Oberfläche mit den übrigen Alkenyl- oder Alkinyl-Gruppen; und
(g2) Bestrahlen der Oberfläche mit der Thiolverbindung mit UV-Licht.

Schritt (g) bzw. Schritt (g1) kann durch Aufbringen der entsprechenden Thiolverbindung oder einer Lösung davon auf die Oberfläche des Substrats, erhalten aus dem Schritt (f), erfolgen. Vorzugsweise erfolgt die Umsetzung mit einer Lösung der entsprechenden Thiolverbindung, beispielsweise einer 1 bis 40 vol%-igen Lösung, vorzugsweise einer 5 bis 20 vol%-igen Lösung und am meisten bevorzugt einer 10 vol%-igen Lösung. Das Reaktionsmedium (Medium) für Schritt (g) unterliegt dabei keinen besonderen Beschränkungen. Beispielsweise kann es sich bei dem Medium um ein wässriges Medium, ein Medium auf Basis eines oder mehrerer organischer Lösungsmittel oder um ein Medium auf Basis eines Gemisches aus Wasser und einem oder mehreren organischen Lösungsmitteln handeln. Vorzugsweise wird das Reaktionsmedium für die Umsetzung gemäß (i) in Schritt (g) des erfindungsgemäßen Herstellungsverfahrens ausgewählt aus der Gruppe, bestehend aus Wasser und Ethanol. Am bevorzugtesten ist das Reaktionsmedium für die Umsetzung gemäß (i) in Schritt (g) des erfindungsgemäßen Herstellungsverfahrens ein Gemisch aus Wasser und Ethanol, vorzugsweise im Volumenverhältnis 1:1. Vorzugsweise wird das Reaktionsmedium für die Umsetzung gemäß (ii) in Schritt (g) des erfindungsgemäßen Herstellungsverfahrens ausgewählt aus der Gruppe, bestehend aus Aceton und Dichlormethan. Am bevorzugtesten ist das Reaktionsmedium für die Umsetzung gemäß (ii) in Schritt (g) des erfindungsgemäßen Herstellungsverfahrens Aceton.

In einer bevorzugten Ausführungsform des erfindungsgemäßen Herstellungsverfahrens erfolgt Schritt (g) in Gegenwart eines Photoinitiators, wie beispielsweise 2,2-Dimethoxy-2-phenylacetophenon (DMPAP). Der Photoinitiator kann in Schritt (g) beispielsweise in einer Konzentration von 1,0 bis 30 mg mL⁻¹, insbesondere von 1,0 bis 20 mg mL⁻¹, und am meisten bevorzugt von 5,0 bis 15 mg mL⁻¹ neben der entsprechenden Thiolverbindung vorliegen. Der Photoinitiator kann gemeinsam mit der entsprechenden Thiolverbindung aufgebracht werden.

Die Lichtquelle für Schritt (g2) unterliegt keinen besonderen Beschränkungen. Es kann beispielsweise mit Licht einer Wellenlänge von 200 bis 360 nm, vorzugsweise 260 nm, bei einer Intensität von 0,5 bis 20 mW cm⁻², vorzugsweise von 2 bis 5 mW cm⁻², bestrahlt werden.

Die (Reaktions-)Temperatur im Schritt (g) bzw. Schritt (g2) unterliegt keinen besonderen Beschränkungen. Es können somit übliche Temperaturen vom Fachmann verwendet werden. In einer bevorzugten Ausführungsform ist die (Reaktions-)Temperatur im Schritt (g) bzw. Schritt (g2) von 0 bis 80°C, noch bevorzugter von 10 bis 60°C, noch bevorzugter von 15 bis 40°C, und am meisten bevorzugt von 20 bis 30°C.

Die (Reaktions-)Zeit im Schritt (g) bzw. Schritt (g2) unterliegt keinen besonderen Beschränkungen. Es können somit übliche Dauern vom Fachmann angesetzt werden. In einer bevorzugten Ausführungsform ist die (Reaktions-)Zeit im Schritt (g) bzw. Schritt (g2) von 10 sec bis 40 min, noch bevorzugter von 30 sec bis 15 min, und am meisten bevorzugt von 1 min bis 3 min.

Nach jedem der Schritte (a) bis (g) kann das Substrat gereinigt werden. Die Art der Reinigung unterliegt keinen besonderen Beschränkungen, solange das Substrat und die Dendrimerstrukturen nicht beeinträchtigt werden. Beispielsweise kann das gemusterte Substrat ein- oder mehrmals mit einem oder unterschiedlichen Lösungsmitteln, wie bspw. Aceton und Ethanol, gewaschen werden und anschließend, bspw. mit Druckluft oder an der Umgebungsluft, getrocknet werden. Insbesondere ist es vorteilhaft das Substrat zwischen den Schritten (e) und (f) und/oder zwischen den Schritten (f) und (g) zu reinigen.

Ein weiterer Aspekt der vorliegenden Erfindung betrifft insbesondere die Verwendung eines erfindungsgemäßen gemusterten Substrats für die chemische Synthese eines chemischen Syntheseproduktes, als Charakterisierungsplattform und/oder als Plattform zur Zellbehandlung/Zellkultivierung. Die vorstehenden Definitionen und Ausführungsformen gelten analog für diesen Aspekt der vorliegenden Erfindung. Das erfindungsgemäße gemusterte Substrat eignet sich für die Kombination von chemischer Synthese, analytischer Charakterisierung und Zellbehandlung/Zellkultivierung oder jeweils nur für einen (oder zwei in beliebiger Kombination) der drei Teilbereiche. In einer bevorzugten Ausführungsform der erfindungsgemäßen Verwendung wird das gemusterte Substrat zunächst für die chemische Synthese eines chemischen Syntheseproduktes und anschließend als Charakterisierungsplattform zur Charakterisierung des chemischen Syntheseprodukts und/oder für die Behandlung von mindestens einer Zelle mit dem chemischen Syntheseprodukt auf dem gemusterten Substrat verwendet.

Die chemische Synthese unterliegt keinen besonderen Beschränkungen. Der Fachmann kann somit die üblichen Methoden verwenden. Das Auftragen der Substanzen (als Reinstoff oder in Lösung) kann beispielsweise mittels eines Dispensers, durch diskontinuierliche Benetzung, bei der ein großer Tropfen einer Lösung über die Oberfläche geführt wird unter spontaner Ausbildung kleinerer Mikrotropfen auf den ersten Bereichen, oder manuelles Pipettieren erfolgen. Lösungsmittel können beispielsweise unter vermindertem Druck entfernt und durch die oben genannten Auftragsmittel hinzugefügt werden. Lösungsmittel und Reagenzien können auch durch Sandwichen mit einem zweiten gemusterten Substrat hinzugefügt und entfernt werden. Hierfür wird auf einem zweiten gemusterten Substrat auf den ersten Bereichen eine Lösung aufgetragen und das zweite Substrat mit dem ersten Substrat übereinander gelegt, sodass die Tröpfchen auf den ersten Bereichen des zweiten Substrats mit den ersten Bereichen des ersten Substrats in Kontakt gebracht werden.

Die Charakterisierungsverfahren, die bei Verwendung des erfindungsgemäßen gemusterten Substrats als Charakterisierungsplattform, angewandt werden können, unterliegen keinen besonderen Beschränkungen. Es können also herkömmliche Analyseverfahren angewandt werden. Insbesondere ist es vorteilhaft Analyseverfahren anzuwenden, die ein elektrisch leitfähiges Substrat benötigen, wie bspw. MALDI-TOF MS und Rasterelektronenmikroskopie, da das erfindungsgemäße gemusterte Substrat ein solches bereitstellen kann ohne die Notwendigkeit eines Probentransfers. Lösungsmittel, Reagenzien etc. können bspw. wie oben beschrieben aufgebracht und entfernt werden.

Die Zellbehandlungsverfahren (vorzugsweise in vitro) unterliegen keinen besonderen Beschränkungen und sind dem Fachmann bekannt. Beispiele für entspreche Zellbehandlungsverfahren sind Toxizitätsscreening, Transformationsverfahren, Transfektionsverfahren und Wirkstoffscreenings. Zellen, Zellsuspensionen, Lösungsmittel, Reagenzien etc. können bspw. wie oben beschrieben aufgebracht und entfernt werden. Prinzipiell sind auch in vivo Behandlungsverfahren möglich. Es können beispielsweise Tröpfchen mit Fisch(embryonen) (z.B. Zebrafisch oder Medaka) erzeugt werden, diese mit einem chemischen Syntheseprodukt behandelt und die Auswirkungen dann untersucht werden.

In einem Zellbehandlungsverfahren können beispielsweise Tröpfchenarrays mit einer Zellsuspension erzeugt werden und die Zellen dann auf dem erfindungsgemäßen gemusterten Substrat kultiviert werden. Die Zellen könnten dann auch mit Wirkstoffen behandelt werden, die nicht auf dem erfindungsgemäßen gemusterten Substrat synthetisiert wurden. Außerdem kann beispielsweise eine zu behandelnde Zellsuspension auf die einzelnen ersten Bereiche des erfindungsgemäßen gemusterten Substrats gegeben werden, die zuvor darauf hergestellte chemische Syntheseprodukte aufweisen. Davor kann das Lösungsmittel, in dem die chemischen Syntheseprodukte vorliegen, entfernt werden, um bspw. etwaige negative Auswirkungen auf die Zellen zu vermeiden. Anschließend kann auf dem erfindungsgemäß gemusterten Substrat eine Zellsuspension (in vitro und/oder in vivo) durch die oben beschriebenen Methoden aufgetragen werden. Die Zellsuspension kann mit dem Fachmann geläufigen Zusätzen behandelt werden.

Durch die vorliegende Erfindung wird es durch eine Oberflächenmodifizierung von Substraten (beispielsweise Glas-Objektträgern o.ä.) und deren anschließende Oberflächenmusterung vorzugweise ermöglicht, eine Plattform zu erzeugen, deren Oberfläche vorzugsweise stark hydrophile und gleichzeitig omniphile Spots aufweist, die durch vorzugsweise stark hydrophobe und gleichzeitig omniphobe Bereiche voneinander abgegrenzt sind. Diese neue Oberflächenfunktionalisierung ermöglicht vorzugsweise die Ausbildung von Tröpfchen-Arrays mit Lösungen mit geringer und hoher Oberflächenspannung, und ist somit dazu geeignet, Tröpfchen-Arrays mit organischen Lösungsmittel für chemische Synthesen und Tröpfchen-Arrays mit wässrigen Lösungsmitteln für (zell-)biologische Screenings zu erzeugen.

Die aktive Arbeitszeit zur Herstellung von bspw. Poly(thioether) Dendrimeren der Generation 3 (G3) inklusive anschließender Musterung beträgt lediglich etwa 15 Minuten. Somit handelt es sich um eine schnelle Methode, die zusätzlich einfach zu parallelisieren und automatisieren ist. Die Materialkosten von bspw. Poly(thioether) Dendrimer-Slides (im Folgenden kurz Dendrimer-Slides) sind im Vergleich zu den Materialkosten von herkömmlichen HSTL-Slides signifikant geringer.

Da es sich bei der Oberflächenmodifizierung des erfindungsgemäßen gemusterten Substrats um eine kovalente Modifizierung handelt, weißt die Oberfläche des Substrats im Gegensatz zu bspw. HSTL-Slides eine hohe mechanische Stabilität auf. Das Auftragen einer zusätzlichen Polymerschicht (wie bei HSTL Slides benötigt) ist nicht mehr notwendig, wodurch zusätzliche Arbeitsschritte gespart werden, der Herstellungs-Prozess vereinfacht, die Arbeitszeit verkürzt, und somit die Herstellungskosten zunehmend minimiert werden. Durch das Fehlen der PolymerSchicht sind bspw. entsprechende Dendrimer-Slides zu jeder Zeit transparent und können daher auch mittels visueller Analysemethoden (wie beispielsweise (Durchlicht-)Mikroskopie) untersucht werden. Das erfindungsgemäße gemusterte Substrat kann außerdem vorzugsweise gereinigt und ohne Verlust der Oberflächenmusterung wiederverwendet werden.

In der Syntheseplanung muss mit dem erfindungsgemäßen gemusterten Substrat nicht mehr berücksichtigt werden, welcher Slide-Typ verwendet werden muss. Mit den entsprechenden Dendrimer-Slides existiert nunmehr ein Slide-Typ, der vorzugsweise mit allen Lösungsmitteln kompatibel ist. Im Vergleich zum Stand der Technik werden nicht mehr länger zwei verschiedene Slide-Typen (LSTL und HSTL) benötigt, um die Prozesse der chemischen Synthese mit bspw. biologischem Screening zu kombinieren. Dendrimere Slides ermöglichen in miniaturisierter und parallelisierter Form kombinatorische Synthese in flüssiger Phase im Array-Format, aber auch Festphase-Synthese wird vorzugweise unterstützt, wobei die Ausbeute durch eine gesteigerte Oberflächen-Beladungskapazität im Vergleich zu LSTL Slides vorzugsweise erhöht wird. Aufgrund vorzugsweise fehlender physikalischer Grenzen zwischen den Spots - wie es etwa bei Mikrotiterplatten der Fall ist - kann das Array extrem miniaturisiert werden. Da sich die Tröpfchen vorzugsweise alle auf einer Ebene, ohne Vertiefungen befinden, kann das komplette Array in einem einzigen Schritt weiter umgesetzt werden, indem es bspw. mit einem weiteren Array in Kontakt gebracht wird. Im Gegensatz zu Mikrotiterplatten-Ansätzen müssen die einzelnen Substanzen dabei nicht aus den jeweiligen *wells* heraus pipettiert werden.

Das erfindungsgemäße gemusterte Substrat ermöglicht vorzugsweise erstmals eine direkte Verbindung zwischen chemischer Synthese einer Molekül-Bibliothek mit der nachfolgenden zellbiologischen Auswertung im selben Array, auf nur einem einzigen Substrat, insbesondere Slide. Nach vollendeter Synthese kann das (organische) Lösungsmittel (mit geringer Oberflächenspannung) verdampft werden und direkt im Anschluss im gleichen Array, auf demselben Substrat, ein Tröpfchen-Array mit Zellsuspension erstellt werden. Ein Transfer der synthetisierten Verbindungen auf eine andere Plattform, die zellkompatibel ist, ist nicht mehr notwendig, wodurch Arbeitsschritte und Materialien gespart werden, was wiederum die Kosten zur Durchführung der Experimente mindert. Die neue Substrat-Oberfläche weißt vorzugsweise ausgezeichnete Kompatibilität mit Zellen auf (sehr hohe Zellviabilität).

Ferner wird es durch erfindungsgemäßen gemusterten Substrats vorzugsweise ermöglicht konduktive Oberflächen (z.B. Indium-Zinnoxid-beschichtete Oberflächen) unter Erhalt der Konduktivität zu mustern, sodass auf dem flachen Substrat ein Tröpfchen-Array mit Lösungen mit geringer und großer Oberflächenspannung ausgebildet werden kann. Diese Eigenschaft ermöglicht, dass die Plattform zusätzlich in analytische Methoden angewendet werden kann, die eine elektrisch leitfähige Oberfläche der Plattform benötigen, wie bspw. MALDI-TOF MS und SEM. Ein Transfer zwischen Synthese- und Charakterisierungs-Plattform ist vorzugsweise nicht mehr notwendig.

Das erfindungsgemäße gemusterte Substrat vereint vorzugsweise sämtliche Bereiche der frühen Wirkstoffentwicklung im miniaturisierten und parallelisierten Array-Format. Sowohl kombinatorische Synthese in flüssiger (aber auch an fester) Phase, analytische Charakterisierung (bspw. durch MALDI-TOF MS, SEM oder Sekundärionen-Massenspektrometrie (ToF-SIMS)), und biologisches Screening (bspw. in Zellexperimenten) werden auf einem einzigen Slide vorzugsweise ermöglicht. Ein Transfer der Substanzen zwischen den einzelnen Bereichen ist nicht mehr notwendig. Das Zuführen zusätzlicher Reagenzien / Zellsuspension / etc. und das Entfernen einzelner oder sämtlicher Tröpfchen ist durch die vorzugsweise offene und flache Struktur des Tröpfchen-Arrays besonders einfach möglich durch bspw. das in Kontakt bringen mit einem weiteren Array, manuelles Pipettieren oder durch Dispenser-Systeme.

Die Figuren zeigen:
Fig. 1: Schematische Darstellung der chemischen Struktur eines Poly(thioether)-Dendrimers der dritten Generation ausgehend von einem Kern (G0).

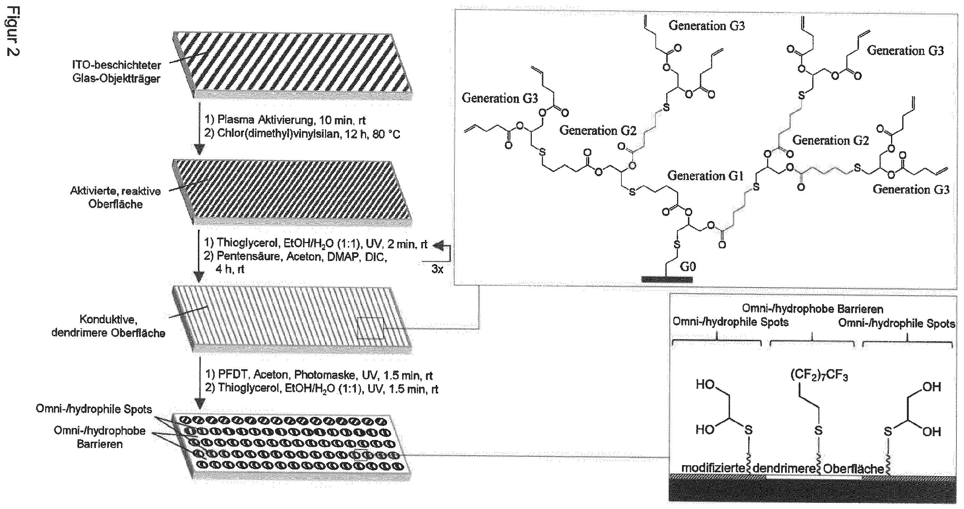

Fig. 2: Herstellungsprozess gemusterter, dendrimerer Oberflächen. Eine GlasOberfläche (oder Glas-ähnliche Oberfläche wie beispielsweise Indium-Zinnoxid (ITO)) wird zunächst aktiviert und mit Chlor(dimethyl)vinylsilan modifiziert. Die dendrimere Struktur wird anschließend in einer repetitiven, photochemischen Thiol-En Click Reaktion mit Thioglycerol, gefolgt von einer Veresterung mit 4-Pentensäure aufgebaut. Die Musterung der Oberfläche erfolgt mit 1*H*,1*H*,2*H*,2*H*-Perfluordecanthiol (PFDT) und Thioglycerol zur Erzeugung hydrophiler/omniphiler Spots, die durch hydrophobe/omniphobe Barrieren voneinander abgegrenzt sind.
Fig. 3: Reaktionszyklus zum Aufbau dendrimerer Strukturen an einer Oberfläche. Der Zyklus besteht aus einer photochemischen Thiol-En-Click-Reaktion mit Thioglycerol (Verbindung 2), gefolgt von einer Veresterung mit 4-Pentensäure (Verbindung 4).
Fig. 4: Messung der statischen Wasser-Kontaktwinkel auf PFDT- und Cysteamin-modifizierten Bereichen auf Dendrimer-Slides der Generation G0 (entspricht konventionellen LSTL Slides) bis Generation G4.
Fig. 5: Vergleich des Fortschreitwinkels (*θ*_{adv}) und des Rückzugwinkels (*θ*_{rec}) von Wassertröpfchen auf PFDT-modifizierten und Cysteamin-modifizierten Bereichen zwischen konventionellen Slides (LSTL Slides bzw. G0 Slides) mit Dendrimer Slides der Generation G3.
Fig. 6: Tröpfchen verschiedener Lösungsmittel (geringe Oberflächenspannung (Ethanol) bis große Oberflächenspannung (Wasser) von links nach rechts) auf gemustertem Dendrimer-Slide.
Fig. 7: Vergleich der Wasserkontaktwinkel verschieden modifizierter Dendrimer Slides, die durch verschiedene Methoden (Thiol-En- und Thiol-In-Chemie) hergestellt wurden.
Fig. 8: Messung des elektrischen Stroms an vier verschiedenen Punkten jeweils im Abstand von 1 cm) auf der Oberfläche von Dendrimer-Slides.
Fig. 9a-d: Ergebnisse des Zellviabilität-Screenings. (a) Alle drei getesteten Zelllinien (HeLa, HEK293T und Jurkat) zeigen auf Spots der Dendrimer-Slides eine ausgezeichnete Zellviabilität von über 90%. (b) Mikroskopie-Bild von HeLa Zellen (Viabilität: 92,5±3,3%) auf Spots von Dendrimer-Slides. (c) Mikroskopie-Bild von HEK293T Zellen (Viabilität: 98,2±0,2%) auf Spots von Dendrimer-Slides. (d) Mikroskopie-Bild von Jurkat Zellen (Viabilität: 99,2±0,1%) auf Spots von Dendrimer-Slides. Maßstableiste: 50 µm.
Fig. 10: Tröpfchen-Array und fluoreszenzmikroskopische Aufnahme im Propidiumiodid-Kanal von gereinigten Dendrimer-Slides nach Zellexperimenten. Zur einfacheren Visualisierung wurde von der fluoreszenzmikroskopischen Aufnahme in nachträglicher Bildbearbeitung ein Negativ-Bild erzeugt. Nach mechanischer Reinigung mittels Zahnbürste, Schwamm und Seife konnten keine Zelltrümmer mehr auf der Oberfläche beobachtet werden. Auch anschließend können noch TröpfchenArrays erzeugt werden.
Fig. 11: 3-Komponenten-Reaktion zur Synthese Lipid-ähnlicher Moleküle (Lipidoide) und die hierzu verwendeten Vorläufer-Moleküle. Zur Synthese wurden auf den Spots des Arrays (rund, Durchmesser 2,83 mm) je 1,5 µL Amin-Komponente und 1,5 µL Thiolacton-Pyridyldisulfid-Lösung mittels Dispenser miteinander kombiniert.
Fig. 12: Auswertung des Toxizitäts-Screenings. Dargestellt ist die Zellviabilität in [%] nach 24 h Inkubation von HEK293T Zellen, die auf einer zuvor synthetisierten Lipidoid-Bibliothek ausgesät wurden.

Die vorliegende Erfindung wird anhand der folgenden, nicht-einschränkenden Beispiele näher erläutert.

### Allgemeines

### Allgemeine Arbeitsvorschriften:

### Aktivierung und Silanisierung von Slides (Trägern):

Zur Aktivierung von Glasträgern (bspw. normalem Siliziumoxid-Glasträger (Schott Glas) und Indium-Zinnoxid-beschichteten Glas-Trägern (ITO Slides, Bruker)) wurden diese für 10 Minuten bei Raumtemperatur mittels Plasma Behandlung gereinigt. Die Silanisierung der Oberfläche der Glasträger erfolgte in Gasphase mit 100 µL Chlor(dimethyl)vinylsilan (Sigma-Aldrich) für 10 Stunden bei 80 °C unter Vakuum (50 mbar). Die Oberfläche wurde zunächst mit Aceton, anschließend mit Ethanol gespült und mit Druckluft getrocknet.

### Allgemeine Arbeitsvorschrift eines Zyklus zur Generierung einer Verzweigung der an einer glatten Oberfläche aufgebauten Dendrimer-Struktur eine Generation mit 4-Pentensäure:

Auf der Oberfläche von zuvor silanisierten Slides wurden 250 µL einer 10 vol% 1-Thioglycerol-Lösung (in Ethanol/H₂O (1:1), 10 mg mL⁻¹ 2,2-Dimethoxy-2-phenylacetophenon (DMPAP, Sigma-Aldrich); Sigma-Aldrich) aufgetragen. Anschließend wurde die Lösung mit einem Quarz-Glas abgedeckt und der Slide für 2 Minuten bei Raumtemperatur mit UV-Licht der Wellenlänge 260 nm (OAI model 30) bei einer Intensität von 3 mW cm⁻² belichtet. Die Oberfläche wurde mit Ethanol gespült und mit Druckluft getrocknet. Der Slide wurde unter Rühren für 4 Stunden in eine auf -20°C gekühlte Veresterungslösung (45 mL Aceton (Merck), 56 mg 4-(Dimethylamino)pyridin (DMAP, Novabiochem), 125 µL 4-Pentensäure (Sigma-Aldrich) und 180 µL N,N'-Diisopropylcarbodiimin (DIC, Alfa Aesar)) eingelegt und die Temperatur der Reaktionslösung dann langsam auf Raumtemperatur erhöht. Die Oberfläche wurde zunächst mit Aceton, anschließend mit Ethanol gewaschen und mit Druckluft getrocknet.

### Allgemeine Arbeitsvorschrift eines Zyklus zur Generierung einer Verzweigung der an einer glatten Oberfläche aufgebauten Dendrimer-Struktur eine Generation mit 4-Pentinsäure:

Auf der Oberfläche von zuvor silanisierten Slides wurden 250 µL einer 10 vol% 1-Thioglycerol-Lösung (in Ethanol/H₂O (1:1), 10 mg mL⁻¹ 2,2-Dimethoxy-2-phenylacetophenon (DMPAP, Sigma-Aldrich); Sigma-Aldrich) aufgetragen. Anschließend wurde die Lösung mit einem Quarz-Glas abgedeckt und der Slide für 2 Minuten bei Raumtemperatur mit UV-Licht der Wellenlänge 260 nm (OAI model 30) bei einer Intensität von 3 mW cm⁻² belichtet. Die Oberfläche wurde mit Ethanol gespült und mit Druckluft getrocknet. Der Slide wurde unter Rühren für 4 Stunden in eine auf -20°C gekühlte Veresterungslösung (45 mL Aceton (Merck), 56 mg 4-(Dimethylamino)pyridin (DMAP, Novabiochem), 111,6 mg 4-Pentinsäure (Sigma-Aldrich) und 180 µL N,N'-Diisopropylcarbodiimin (DIC, Alfa Aesar)) eingelegt und die Temperatur der Reaktionslösung dann langsam auf Raumtemperatur erhöht. Die Oberfläche wurde zunächst mit Aceton, anschließend mit Ethanol gewaschen und mit Druckluft getrocknet.

### Musterung der Oberfläche mit Perfluordecanthiol und 1-Thioglycerol:

300 µL einer 20 vol% 1*H*, 1*H*, 2*H*, 2*H*-Perfluordecanthiol-Lösung (10 mg mL⁻¹ 2,2-Dimethoxy-2-phenylacetophenon (DMPAP, Sigma-Aldrich); Sigma-Aldrich) in Aceton (Merck) wurden auf einer zuvor modifizierten Oberfläche aufgetragen und durch eine Fotomaske (Rose Fotomasken) für 1,5 Minuten bei Raumtemperatur mit UV-Licht der Wellenlänge 260 nm (OAI model 30) bei einer Intensität von 3 mW cm⁻² belichtet. Die Oberfläche wurde mit Aceton gespült und mit Druckluft getrocknet. 300 µL einer 10 vol% 1-Thioglycerol-Lösung (in Ethanol/H₂O (1:1), 10 mg mL⁻¹ 2,2-Dimethoxy-2-phenylacetophenon (DMPAP, Sigma-Aldrich); Sigma Aldrich) wurden auf der Oberfläche aufgetragen und durch ein Quarz-Glas für 1,5 Minuten bei Raumtemperatur mit UV-Licht der Wellenläng 260 nm (OAI model 30) bei einer Intensität von 3 mW cm⁻² belichtet. Die Oberfläche wurde mit Ethanol gespült und mit Druckluft getrocknet.

### Musterung der Oberfläche mit Perfluordecanthiol und Cysteamin-Hydrochlorid:

300 µL einer 20 vol% 1*H*, 1*H*, 2*H*, 2*H*-Perfluordecanthiol-Lösung (10 mg mL⁻¹ 2,2-Dimethoxy-2-phenylacetophenon (DMPAP, Sigma-Aldrich); Sigma-Aldrich) in Aceton (Merck) wurden auf einer zuvor modifizierten Oberfläche aufgetragen und durch eine Fotomaske (Rose Fotomasken) für 1,5 Minuten bei Raumtemperatur mit UV-Licht der Wellenlänge 260 nm (OAI model 30) bei einer Intensität von 3 mW cm⁻² belichtet. Die Oberfläche wurde mit Aceton gespült und mit Druckluft getrocknet. 300 µL einer 10 wt% Cysteamin-Hydrochlorid-Lösung (in Ethanol/H₂O (1:1), 10 mg mL⁻¹ 2,2-Dimethoxy-2-phenylacetophenon (DMPAP, Sigma-Aldrich); Alfa Aesar) wurden auf der Oberfläche aufgetragen und durch ein Quarz-Glas für 1,5 Minuten bei Raumtemperatur mit UV-Licht der Wellenläng 260 nm (OAI model 30) bei einer Intensität von 3 mW cm⁻² belichtet. Die Oberfläche wurde mit Ethanol gespült und mit Druckluft getrocknet.

### Wasser-Kontaktwinkel-Messungen:

Die Oberflächen der Glasträger wurden durch Wasser-Kontaktwinkel-Messungen unter Verwendung des Drop Shape Analyzers DSA25 (Krüss) charakterisiert. Es wurden der Fortschreitwinkel (engl. *advancing angle, θ*_{adv}), der Rückzugswinkel (engl. *receding angle, θ*_{rec}) und der statische Winkel (engl. *static angle, θ*ₛₜₐₜ) gemessen. Hierfür wurden 40 µL deionisiertes Wasser bei einer Geschwindigkeit von 0,3 µL s⁻¹ auf der jeweiligen Oberfläche aufgetragen und wieder abgesaugt, und dabei die entsprechenden Winkel gemessen.

### Beispiel 1: Herstellung von Dendrimer-Slides mit 2-Pentensäure

Die Herstellung eines Dendrimer Slides ist schematisch in Figur 2 dargestellt.

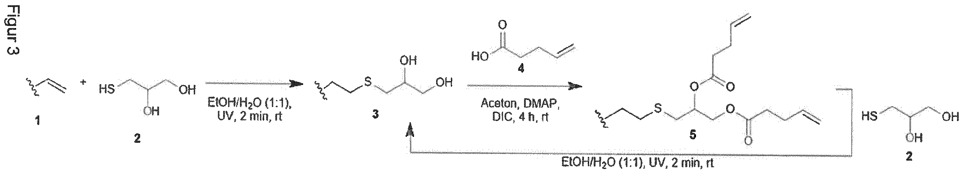

Zur Herstellung von Dendrimer Slides wurden normale Siliziumoxid-Glasträger (Schott Glas) und Indium-Zinnoxid-beschichteten Glas-Träger (ITO Slides, Bruker) verwendet. Zur Aktivierung wurden die Glas-Träger mittels Plasma Behandlung gereinigt und silanisiert, wie in der allgemeinen Arbeitsvorschrift angeben. Die Oberflächen der Träger wurden silanisiert, um diese durch Einbringen von Doppelbindungen (vgl. Struktur 1 in Figur 3) reaktiver zu machen.

Anschließend wurden die Oberflächen weiter funktionalisiert, um darauf dendrimere Strukturen aufzubauen. Figur 3 zeigt die beiden iterativ ablaufenden Syntheseschritte, die einen Zyklus und einen Verzweigungsgrad und somit eine Generation der Oberflächenstruktur ausmachen. Nach der Umsetzung der mit Doppelbindungen funktionalisierten, reaktiven Oberflächen (vgl. Struktur 1 in Figur 3) mit Thioglycerol (vgl. Verbindung 2 in Figur 3) in einem ersten Schritt des Zyklus weisen die Oberflächen zahlreiche Hydroxy-Gruppen (vgl. Struktur 3 in Figur 3) auf, die in dem zweiten Schritt des Zyklus in einer Veresterung mit 4-Pentensäure (vgl. Verbindung 4 in Figur 3) umgesetzt werden. Somit werden die Hydroxy-Endgruppen der Oberflächen in Endgruppen mit Doppelbindungen (vgl. Struktur 5 in Figur 3) umgewandelt. Diese Doppelbindungen können in einem weiteren Zyklus erneut in einer Thiol-En-Reaktion mit Thioglycerol (vgl. Verbindung 2 in Figur 3; rechte Seite), wodurch erneut funktionelle Hydroxy-Endgruppen erzeugt werden, und anschließend mit 4-Pentensäure umgesetzt werden. Die beiden Schritte des Zyklus können weiter wiederholt werden, wobei bei jedem Durchlauf des Zyklus der Verzweigungsgrad um 2ⁿ steigt (n: Anzahl an Zyklen). Die erzeugten Dendrimere können chemisch als Poly(thioether) Dendrimere bezeichnet werden.

Die oben beschriebenen Prozesse wurden auf die Träger angewandt durch Ausführen der allgemeinen Arbeitsvorschrift bezüglich eines Zyklus (eine Generation) zur Generierung einer Verzweigung, der an einer glatten Oberfläche aufgebauten Dendrimer-Struktur, mit 4-Pentensäure. Der Zyklus wurde insgesamt drei Mal durchgeführt, wie bspw. in Figur 2, gezeigt. Nach dem dritten Durchlauf des Zyklus konnte die Anzahl von ursprünglich einer immobilisierten Doppelbindung auf acht Doppelbindungen erhöht werden (vgl. Figur 1).

Diese endständigen Doppelbindungen wurden anschließend in weiteren photochemischen Thiol-En-Click-Reaktionen mit stark hydrophoben (1*H*,1*H*,2*H*,2*H-*Perfluordecanthiol (PFDT)) und hydrophilen (Thioglycerol) Molekülen ortspezifisch durch eine Photomaske oder durch einen Laserstrahl umgesetzt werden. Somit konnten die Oberflächen gemustert werden unter Erhalt von Thioglycerolfunktionalisierten Spots, die durch Grenzen aus PFDT-funktionalisierten Barrieren voneinander abgetrennt sind.

Die oben beschriebenen Endfunktionalisierungen wurden auf die Träger angewandt durch Ausführen der allgemeinen Arbeitsvorschrift bezüglich der Musterung der Oberfläche mit Perfluordecanthiol und 1-Thioglycerol.

Es konnten dabei Spots mit unterschiedlichen Durchmessern, bspw. zwischen 500 µm und 2,83 mm, und beliebiger Geometrie erzeugt werden.

### Beispiel 2: Herstellung von Dendrimer-Slides mit 2-Pentinsäure

Analog zu Beispiel 1 wurde eine dendrimere Oberflächenmodifizierung auch mit 4-Pentinsäure anstatt von 4-Pentensäure durchgeführt. In diesem Fall wird die dendrimere Struktur durch eine Thiol-In-Reaktion aufgebaut und abschließend gemustert. Die repetitiven Einheiten wurden dabei auf den Träger durch Anwendung der allgemeinen Arbeitsvorschrift bezüglich eines Zyklus (eine Generation) zur Generierung einer Verzweigung, der an einer glatten Oberfläche aufgebauten Dendrimer-Struktur, mit 4-Pentinsäure aufgebracht.

### Beispiel 3: Untersuchung des Einflusses des Verzweigungsgrades verschiedener Dendrimer-Generationen auf den statischen Wasser-Kontaktwinkel

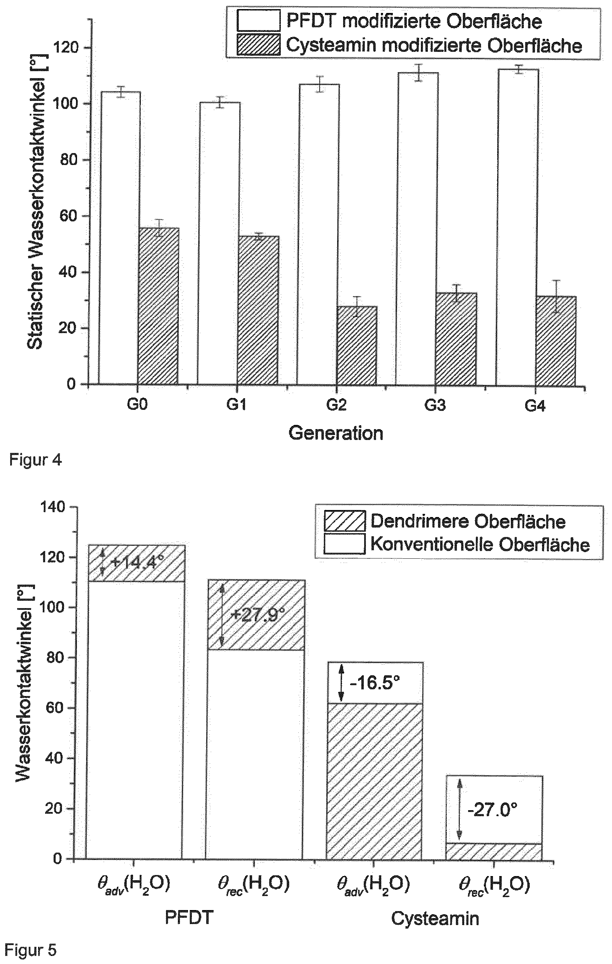

Der Einfluss des Verzweigungsgrades verschiedener Dendrimer-Generationen auf den statischen Wasser-Kontaktwinkel wurde untersucht. Hierfür wurden Dendrimer-Slides hergestellt, die mit 4-Pentensäure verestert wurden. Im letzten Schritt erfolgten Modifikationen der Oberflächen jeweils mit PFDT, um die Eigenschaften der hydrophoben/omniphoben Grenzen (d.h. die zweiten Bereiche) zu charakterisieren, und mit Cysteamin, um die Eigenschaften der hydrophilen/omniphilen Spots (d.h. die ersten Bereiche) zu charakterisieren. Die Ergebnisse sind in Figur 4 wiedergegeben.

Bereits nach der zweiten Generation der Dendrimer-Slides sind eine Zunahme des statischen Kontaktwinkels auf den PFDT-modifizierten Bereichen und eine deutliche Abnahme des statischen Kontaktwinkels auf den Cysteamin-modifizierten Bereichen zu beobachten. Anschließend verändert sich der Kontaktwinkel nur noch wenig. Substrate mit einer dritten Dendrimer-Generation zeigten bspw. statische Kontaktwinkel auf den PFDT-modifizierten Bereichen von *θ*ₛₜₐₜ(H₂O) = 111,5±2,9° und auf den Cysteamin-modifizierten Bereichen von *θ*ₛₜₐₜ(H₂O) = 33,0±3,0°.

### Beispiel 4: Vergleich des Fortschreitwinkels (θ_{adv}) und des Rückzugwinkels (θ_{rec}) von Wassertröpfchen auf PFDT-modifizierten und Cysteamin-modifizierten Bereichen zwischen konventionellen Slides (LSTL Slides bzw. G0 Slides) mit Dendrimer-Slides der Generation G3

Es wurden Fortschreitwinkel (*θ*_{adv}) und Rückzugwinkels (*θ*_{rec}) von Wassertröpfchen auf PFDT-modifizierten und Cysteamin-modifizierten Bereichen von konventionellen Slides (LSTL Slides bzw. G0 Slides) und Dendrimer-Slides der Generation G3 gemessen. Die Ergebnisse sind in Figur 5 wiedergegeben. Beide Winkel wurden durch die dendrimere Oberfläche auf PDFT-modifizierten Bereichen deutlich erhöht und auf Cysteamin-modifizierten Bereichen deutlich verringert. Aufgrund des großen Unterschieds zwischen Rückzugwinkel auf PFDT-modifizierten Bereichen und Cysteamin-modifizierten Bereichen lassen sich Tröpfchen-Arrays auf Dendrimer-Slides sowohl mit Lösungsmittel mit geringer als auch mit hoher Oberflächenspannung erzeugen. Auf konventionellen LSTL Slides ohne dendrimere Oberfläche können jedoch keine Tröpfchen-Arrays gebildet werden. Der Unterschied zwischen dem Rückzugswinkel auf PFDT-modifizierten Bereichen (*θ*_{rec}(H₂O) = 83,3°) und dem Rückzugswinkel auf Cysteamin-modifizierten Bereichen (*θ*_{rec}(H₂O) = 33,7°) ist in konventionellen LSTL Slides deutlich geringer als in erfindungsgemäßen Dendrimer-Slides (vgl. Figur 5).

Die dritte Dendrimer-Generation zeigt einen deutlich größeren Unterschied des Rückzug-Kontaktwinkels (PFDT: *θ*_{rec}(H₂O) = 111,2°; Cysteamin: *θ*_{rec}(H₂O) = 6,7°).

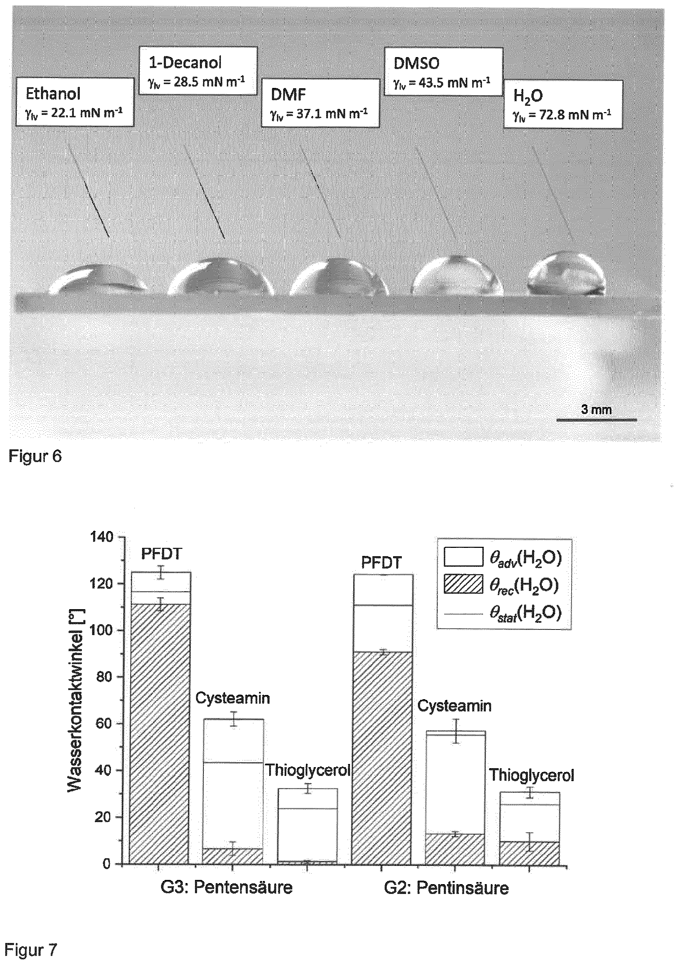

Aufgrund des starken Unterschieds des Rückzugwinkels auf den beiden verschieden modifizierten Bereichen können auf gemusterten, dendrimeren Oberflächen auch Tröpfchen-Arrays mit wässrigen Lösungsmitteln mit hoher Oberflächenspannung erzeugt werden. Da es sich bei der Modifizierung der Oberfläche um eine direkte, kovalente Modifizierung handelt, können aber auch Lösungsmittel mit geringer Oberflächenspannung aufgetragen werden, ohne Diffusion aus den Spots und einer daraus resultierende Kreuzkontamination (vgl. Figur 6).

### Beispiel 5: Vergleich der Wasserkontaktwinkel verschieden modifizierter Dendrimer-Slides, die durch verschiedene Methoden (Thiol-En und Thiol-In Chemie) hergestellt wurden

Es wurden dendrimere Strukturen durch photochemische Thiol-En-Click-Reaktionen unter Verwendung von 4-Pentensäure und durch photochemische Thiol-In-Click-Reaktionen unter Verwendung von 4-Pentinsäure auf unterschiedlichen flachen Substraten erzeugt. Da der Verzweigungsgrad in einer Thiol-In-Reaktion im Vergleich zu einer Thiol-En-Reaktion nochmals verdoppelt wird, wurden die Wasserkontaktwinkel der zweiten Generation durch Thiol-In erzeugter dendrimerer Oberflächen mit der dritten Generation durch Thiol-En erzeugter dendrimerer Oberflächen miteinander verglichen (vgl. Figur 7).

Während die Fortschreitwinkel der mit 4-Pentinsäure hergestellten Dendrimer-Oberflächen auf PFDT-funktionalisierten Bereichen (*θ*_{adv}(H₂O) = 124,2±0,1°) und Cysteamin-funktionalisierten Bereichen (*θ*_{adv}(H₂O) = 57,3±5,2°) nahezu identisch mit den mit 4-Pentensäure hergestellten Dendrimer-Oberflächen sind, ist der Rückzugswinkel der mit 4-Pentinsäure hergestellten Dendrimer-Oberflächen auf den PFDT-funktionalisierten Bereichen (*θ*_{rec}(H₂O) = 91,0±1,1 °) etwas niedriger und auf den Cysteamin-funktionalisierten Bereichen (*θ*_{rec}(H₂O) = 13,2±1,1°) etwas höher als auf den entsprechenden Bereichen mit 4-Pentensäure-Dendrimeren.

Neben den Untersuchungen verschiedener Säurekomponenten zur Synthese der Dendrimere, wurden auch verschiedene Reagenzien zur abschließenden Oberflächenmusterung untersucht (vgl. Figur 7). Eine Funktionalisierung mit Thioglycerol führt zu einer weiteren deutlichen Verbesserung der Hydrophilie/Omniphilie der Oberfläche. Mit Thioglycerol funktionalisierte dendrimere Oberflächen (4-Pentensäure) der dritten Generation weisen einen Fortschreitwinkel von *θ*_{adv}(H₂O) = 32,6±2,2° und einen Rückzugswinkel von *θ*_{rec}(H₂O) = 1,2±0,6 ° auf, was einer Halbierung der entsprechenden Winkel im Vergleich zur Funktionalisierung mit Cysteamin entspricht (vgl. Figur 7).

### Beispiel 6: Modifizierung konduktiver Oberflächen mit Dendrimerstrukturen

ITO-beschichtete Glasobjektträger wurden mit erfindungsgemäßen Dendrimerstrukturen modifiziert. Auf den entsprechenden Substraten konnten ebenfalls Tröpfchen-Arrays mit geringer und großer Oberflächenspannung erzeugt werden.

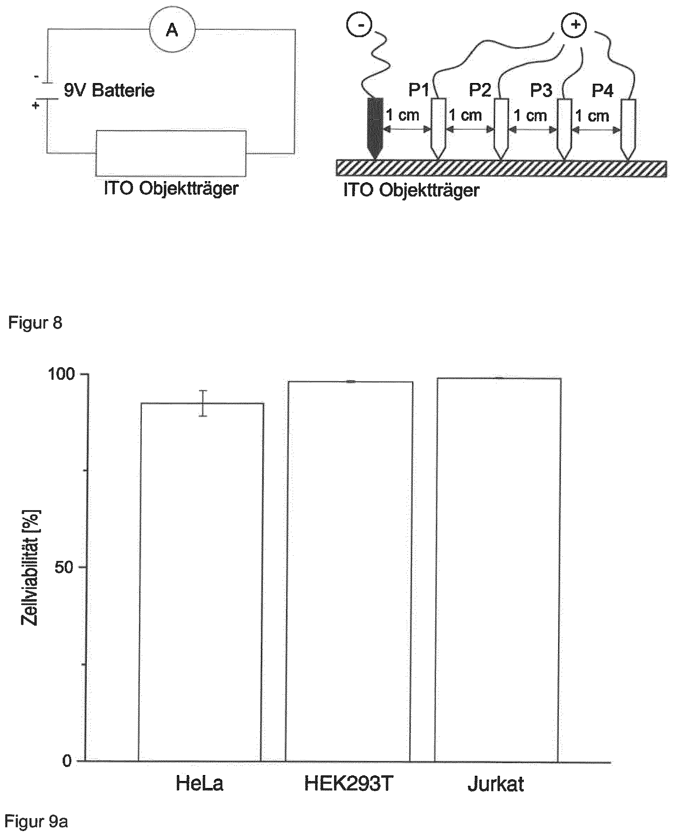

Zur Überprüfung des Erhalts der Leitfähigkeit wurde der elektrische Strom an jeweils vier Positionen (im Abstand von je 1 cm) auf der Oberfläche der modifizierten Glasobjektträger gemessen (vgl. Figur 8). Zur Messung wurde dabei eine 9V Batterie mit einer Rest-Stromstärke von 1,1 A und einer Rest-Spannung von 8,5 V verwendet.

Auf diese Weise wurde nach jedem Schritt in der Herstellung der dendrimeren Oberfläche der elektrische Strom gemessen (vgl. Tabelle 1). Des Weiteren wurde der Einfluss des Lösungsmittels bei den Veresterungsschritten untersucht.

**Tabelle 1: Messung des elektrischen Stroms an vier Positionen (P1-4; vgl. Figur 8) auf Oberflächen nach verschiedenen Schritten in der Herstellung von dendrimeren Slides.**

| **Schritt** | **P1 [A]** | **P2 [A]** | **P3 [A]** | **P4 [A]** | **Lösungsmittel** | **Zeit nach Start [d]** |
|---|---|---|---|---|---|---|
| Unbehandelt (Positiv-Kontrolle) | 0,048 | 0,034 | 0,032 | 0,024 | | 0 |
| Plasma-Aktivierung | 0,042 | 0,035 | 0,031 | 0,023 | | 0 |
| Silanisierung | 0,025 | 0,021 | 0,020 | 0,015 | | 1 |
| 1. Veresterung | 0,024 | 0,021 | 0,015 | 0,011 | Aceton | 1,5 |
| 1. Veresterung | 0,024 | 0,021 | 0,020 | 0,014 | DCM | 1,5 |
| 2. Veresterung | 0,026 | 0,022 | 0,019 | 0,017 | Aceton | 2 |
| 2. Veresterung | 0,025 | 0,022 | 0,019 | 0,017 | DCM | 2 |
| 3. Veresterung | 0,028 | 0,026 | 0,025 | 0,021 | Aceton | 2,5 |
| 3. Veresterung | 0,028 | 0,021 | 0,020 | 0,020 | DCM | 2,5 |
| Gemustert | 0,032 | 0,028 | 0,025 | 0,022 | Aceton | 2,5 |
| Gemustert | 0,027 | 0,024 | 0,021 | 0,018 | DCM | 2,5 |
| Normaler Glas-Slide (Negativ-Kontrolle) | 0 | 0 | 0 | 0 | Aceton | 0 |
| Gemusterter Glas-Slide (G3; Negativ-Kontrolle) | 0 | 0 | 0 | 0 | Aceton | 2,5 |

Es konnte kein Unterschied zwischen der Verwendung von Aceton und Dichlormethan (DCM) festgestellt werden. Der elektrische Strom halbiert sich nach der Silanisierung, bleibt dann jedoch bis zur abschließenden Musterung unverändert erhalten (vgl. Tabelle 1). Es konnten daher konduktive Plattformen erhalten werden. Nach vollendeter on-chip Synthese einen Substanz kann somit auch eine direkte on-chip Charakterisierung mittels MALDI-TOF MS oder anderen Analysemethoden durchgeführt werden, die eine elektrisch leitfähige Plattform benötigen.

### Beispiel 7: Zellviabilitätsscreening auf Dendrimer-Slides

Zellviabilitätsscreenings mit verschiedenen Zelllinien (Adhärente Zellen: HeLa, HEK293T; Suspension-Zellen: Jurkat) wurden zur Evaluierung der Kompatibilität der Oberfläche der erfindungsgemäßen gemusterten Substrate mit (zell-)biologischen Experimenten durchgeführt.

Dabei wurden HeLa und HEK293T Zellen in Dulbecco's Modified Eagle Medium (DMEM, Life Technologies) mit 15% v/v fötalem Kälberserum (FBS; PAA Laboratories) und 1% v/v Penicillin-Streptomycin (Life Technologies) kultiviert. Jurkat Zellen wurden in RPMI 1640 Medium (Gibco) mit 15% v/v FBS (PAA Laboratories) und 1% v/v Penicillin-Streptomycin (Life Technologies) kultiviert.

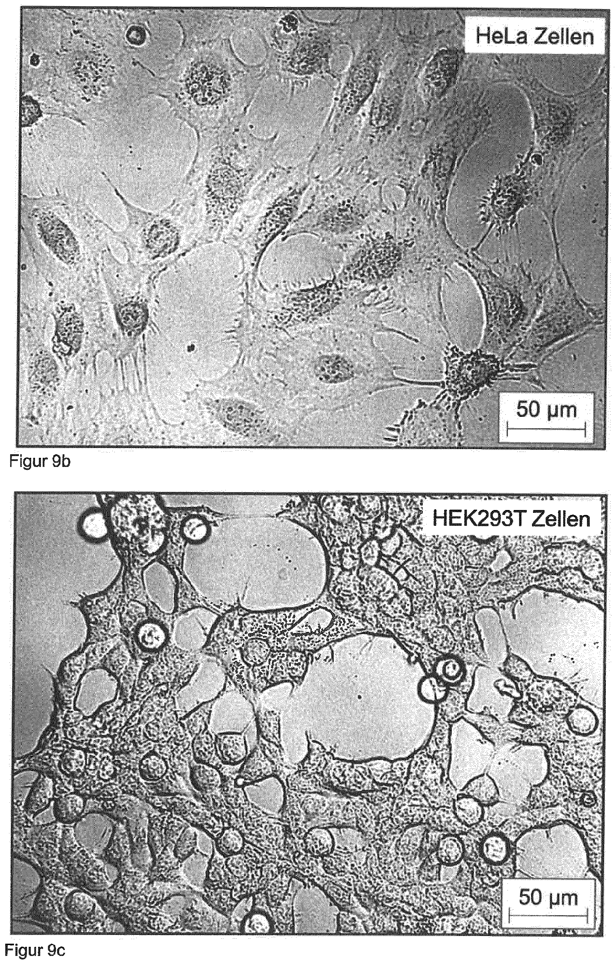

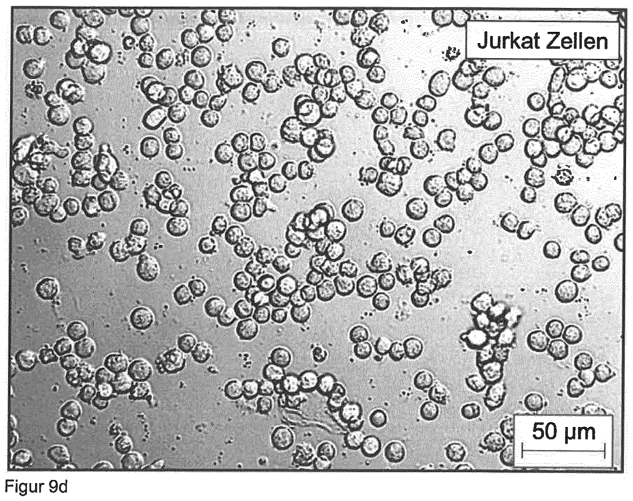

Pro Spot des Substrats wurden 3 µL einer HeLa- und HEK293T-Zellsuspension mit einer Konzentration von je 50.000 Zellen/mL und 3 µL einer Jurkat-Zellsuspension mit einer Konzentration von 80.000 Zellen/mL mittels Dispenser (I-DOT, Dispendix) aufgetragen. Der Durchmesser der runden Spots betrug 2,83 mm, der Abstand zwischen den Spots 1,67 mm. Nach 24h Kultivierung (bei 37 °C und 5% CO₂-Konzentration) wurden die Zellen mit Hoechst 33342 (1:900; 10 mg mL⁻¹; Invitrogen) zur Bestimmung der Gesamtzellzahl (Färbung der Zellkerne) und Propidiumiodid (PI; 1:1350; 1,00 mg mL⁻¹; Invitrogen) zur Bestimmung der Tot-Zellzahl (Färbung toter Zellen) gefärbt. Die Auswertung erfolgte durch Fluoreszenz-Mikroskopie mittels Keyence BZ9000 (Keyence) und der Software ImageJ (Funktion: Analyze Particles). Die Lebend-Zellzahl wurde aus der Differenz der Gesamtzellzahl und der Tot-Zellzahl berechnet. Die Zellviabilität wurde als Quotient der Lebend-Zellzahl und der Gesamtzellzahl in Prozent [%] angegeben (vgl. Figur 9).

Jede der getesteten Zelllinien zeigte eine sehr gute Zellviabilität (>90%) auf den Spots der Dendrimer Slides (vgl. Figur 9a). Des Weiteren zeigen alle drei getesteten Zelllinien die jeweils typische Morphologie (vgl. Figur 9b-d). Die erfindungsgemäßen gemusterten Substrate sind daher hervorragend für biologische Zellscreenings geeignet.

### Beispiel 8: Wiederverwendbarkeit der Dendrimer-Slides

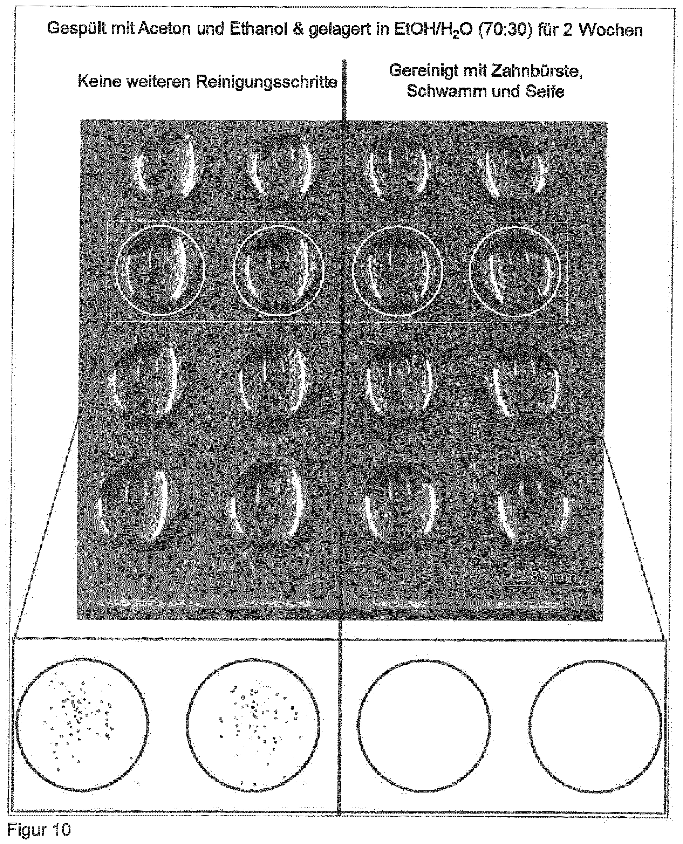

Die Dendrimer Slides aus Beispiel 7, die für die biologischen Zellviabilitäts-Screenings verwendet wurden, wurden gereinigt und darauf getestet, ob sie erneut verwendet werden können. Hierfür wurden die Slides zunächst mit Aceton und Ethanol abgespült, was zur Lyse der noch darauf befindlichen Zellen führte. Eine Trübfärbung der Spots signalisierte dabei das Ausfällen der in den Zellen vorhandenen Proteine. Die Slides wurden zur Sterilisierung zwei Wochen in eine wässrige 70 Vol% Ethanol-Lösung eingelegt. Auch danach war die Trübung der Spots noch zu sehen. Mikroskopische Aufnahmen zeigten noch existierende Zelltrümmer auf der Oberfläche der Spots. Ebenfalls sichtbar durch Fluoreszenz-Mikroskopie war noch die Färbung der toten Zellen durch Propidiumiodid. Teile der Slide-Oberfläche wurden anschließend mechanisch mit Hilfe einer handelsüblichen Zahnbürste und Seife und danach mit einem Schwamm gesäubert. Der Slide wurde nochmals mit Ethanol und Aceton abgespült und mit Druckluft getrocknet. Figur 10 zeigt das Ergebnis des Reinigungsvorgangs.

Eine klare Linie zwischen mechanisch gesäuberter Oberfläche und nicht-gesäuberter Oberfläche ist durch Fluoreszenz-Mikroskopie im Propidiumiodid-Kanal zu beobachten (vgl. Figur 10). Auch mittels Durchlichtmikroskopie im sichtbaren Licht konnten keine Zelltrümmer mehr auf den mechanisch gereinigten Spots der Dendrimer-Oberfläche beobachtet werden. Dennoch konnten Tröpfchen-Arrays auch auf den mechanisch gereinigten Spots erzeugt werden. Es konnte kein Unterschied in Form und Volumen zwischen gereinigten und nicht-gereinigten Spots beobachtet werden (vgl. Figur 10). Die Oberflächen der verwendeten Dendrimer-Slides sind folglich mechanisch stabil und wiederverwendbar. Im Gegensatz dazu würde die Oberfläche von aus dem Stand der Technik bekannten HSTL Slides durch diesen Prozess zerstört werden.

### Beispiel 9: Toxizitätsscreeninq mit Dendrimer-Slides

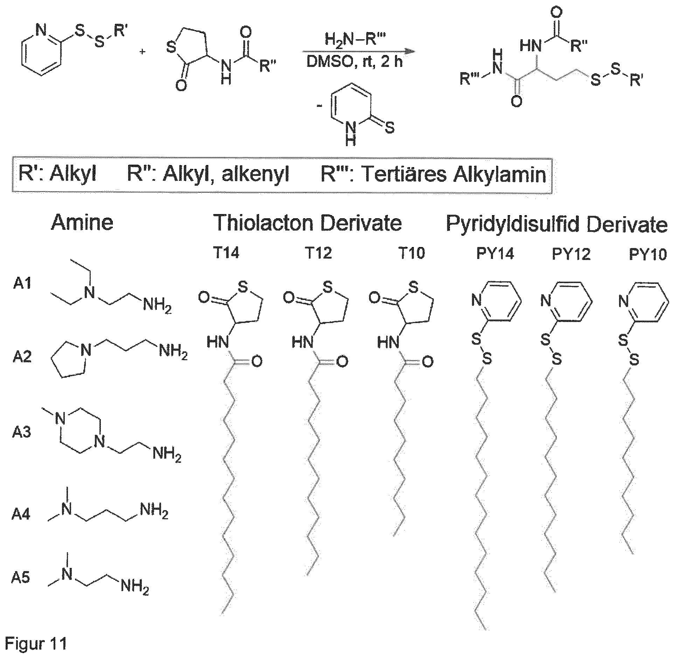

Die Möglichkeit einer chemischen Synthese mit biologischem Screening auf einem einzigen erfindungsgemäßen gemusterten Substrat wurde untersucht. Hierzu wurde zunächst eine Bibliothek aus 25 verschiedenen Lipid-ähnlichen Molekülen (Lipidoiden) kombinatorisch in flüssiger Phase auf einem Dendrimer-Slide (runde Spots, Durchmesser 2,83 mm, Barrieren-Durchmesser 1,67 mm) synthetisiert. Die Synthese basiert auf einer Drei-Komponenten-Reaktion, bei der ein primäres Amin (A1-A5) die Reaktion durch Ringöffnung eines Thiolactons (T10/T12/T14) initiiert und mit dem Disulfidaustausch mit einem Pyridyldisulfid (PY10/PY12/PY14) abschließt. Die verwendeten Vorläufer-Moleküle sind in Figur 11 dargestellt.

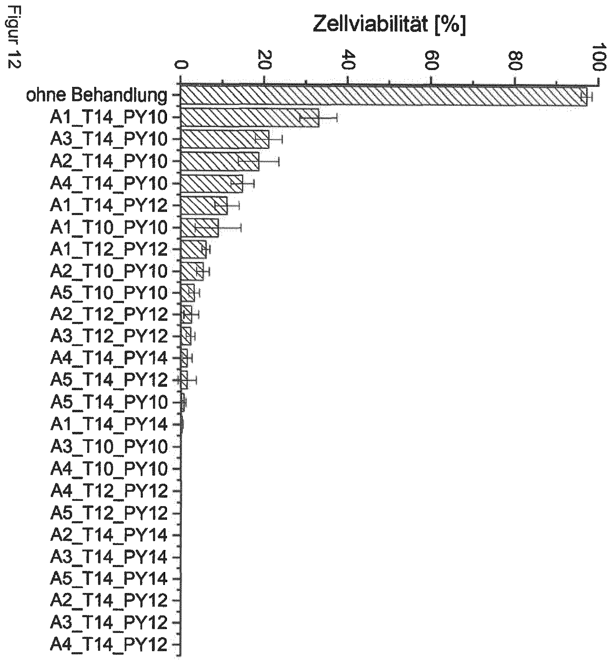

Zur kombinatorischen Synthese der Lipidoid-Bibliothek wurde je 1,5 µL der Amin-Komponente (gelöst in DMSO; 1:24 v/v) (A1-A5) und 1,5 µL einer Mischung (gelöst in DMSO) aus Thiolacton-Derivat (1,67 mg mL⁻¹) und Pyridyldisulfid-Derivat (1,75 mg mL⁻¹) (Mischungen: T10_PY10 / T12_PY12 / T14_PY14 / T14_PY12 / T14_PY10) miteinander vereint. Die Lösungen wurden mittels Dispensers (I-DOT, Dispendix) in kombinatorischem Format aufgetragen. Nach 2 h Reaktionszeit bei Raumtemperatur wurde das Lösungsmittel unter vermindertem Druck entfernt. Zur Bestimmung der Toxizität der Syntheseprodukte wurde anschließend mittels Dispensers 3 µL einer HEK293T-Zellsuspension (50.000 Zellen/mL) auf jeden Spot des Arrays gedruckt. Der Slide wurde für 24 h bei 37 °C und 5% CO₂-Konzentration inkubiert und die Zellen anschließend mit Hoechst 33342 (1:900; 10 mg mL⁻¹; Invitrogen) zur Bestimmung der Gesamtzellzahl (Färbung der Zellkerne) und Propidiumiodid (Pl; 1:1350; 1,00 mg mL⁻¹; Invitrogen) zur Bestimmung der Tot-Zellzahl (Färbung toter Zellen) gefärbt. Die Auswertung erfolgte durch Fluoreszenz-Mikroskopie (Keyence BZ9000 (Keyence)) und der Software ImageJ (Funktion: Analyze Particles). Die Lebend-Zellzahl wurde aus der Differenz der Gesamtzellzahl und der Tot-Zellzahl berechnet. Die Zellviabilität wurde als Quotient der Lebend-Zellzahl und der Gesamtzellzahl in Prozent [%] angegeben (vgl. Figur 12).

Wie in Figur 12 zu erkennen ist, stellten sich sämtliche Substanzen in der getesteten Konzentration als sehr toxisch heraus. Mit einer Zellviabilität von 33,0±4,4% war das Lipidoid A1_T14_PY10 am wenigsten toxisch. Für die Negativkontrolle mit unbehandelten Zellen konnte erneut eine ausgezeichnete Zellviabilität von 97,2±1,3% beobachtet werden. Dies zeigt eindeutig, dass der toxische Effekt nicht durch die Oberfläche verursacht wurde, und bestätigt die vorangegangenen Zellviabilitätsscreenings aus Beispiel 7. Die Erkenntnisse aus diesem primären Screening können im Folgenden zur Vorgabe einer Leitstruktur für chemische Strukturoptimierungs-Experimente dienen.

Es konnte somit erfolgreich gezeigt werden, dass auf erfindungsgemäßen gemusterten Substraten chemische Synthesen in flüssiger Phase direkt mit biologischen Screenings auf einer einzigen Plattform im Tröpfchen-Array-Format vereint werden können.

## Patentansprüche

1. Gemustertes Substrat umfassend erste Bereiche und zweite Bereiche auf einer Oberfläche des Substrats, wobei
die ersten Bereiche erste Dendrimerstrukturen und die zweiten Bereiche zweite Dendrimerstrukturen aufweisen;
die Dendrimerstrukturen jeweils kovalent mit der Substratoberfläche verbunden sind;
die zweiten Bereiche die ersten Bereiche umschließen;
die zweiten Dendrimerstrukturen mindestens ein Strukturelement aufweisen, das sich von den Strukturelementen der ersten Dendrimerstrukturen unterscheidet; und
die Substratoberfläche Hydroxylgruppen oder Silanolgruppen aufweist.

2. Gemustertes Substrat nach Anspruch 1, wobei die Dendrimerstrukturen mindestens zwei aufeinanderfolgende repetitive Einheiten aufweisen und die repetitiven Einheiten jeweils unabhängig voneinander mindestens zwei Verzweigungsstellen aufweisen.

3. Gemustertes Substrat nach Anspruch 1 oder 2, wobei die Dendrimerstrukturen jeweils eine Alkylen-Silyl-Gruppe aufweisen und die Dendrimerstrukturen mittels der jeweiligen Alkylen-Silyl-Gruppe mit der Substratoberfläche verbunden sind.

4. Gemustertes Substrat nach Anspruch 3, wobei die Alkylen-Silyl-Gruppe eine Struktur der folgenden allgemeinen Formel (1) aufweist 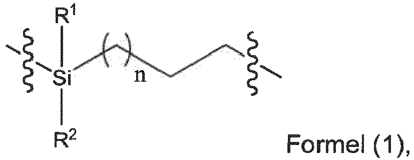
wobei R¹ und R² unabhängig voneinander ausgewählt sind aus der Gruppe, bestehend aus einer Alkylgruppe, einer Cycloalkylgruppe, einer Arylgruppe, einer Heteroarylgruppe, -OA, wobei A unabhängig voneinander ausgewählt ist aus der Gruppe, bestehend aus einer Alkylgruppe, einer Cycloalkylgruppe, einer Arylgruppe und einer Heteroarylgruppe; und
n eine ganze Zahl von 0 bis 6 ist.

5. Gemustertes Substrat nach einem der Ansprüche 2 bis 4, wobei die ersten Dendrimerstrukturen und die zweiten Dendrimerstrukturen jeweils repetitive Einheiten mit der folgenden allgemeinen Formel (2) oder (3) aufweisen 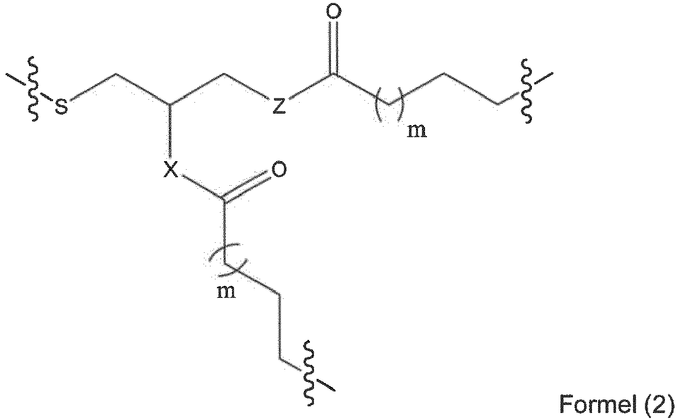 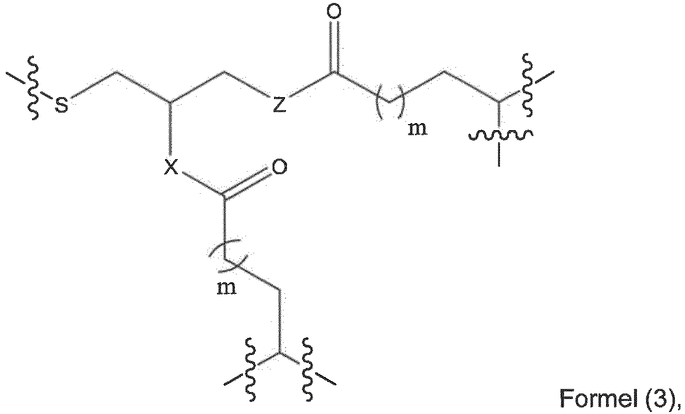
wobei X und Z unabhängig voneinander NR³ oder O sind, wobei jedes R³ unabhängig voneinander ausgewählt ist aus der Gruppe, bestehend aus Wasserstoff, einer Alkylgruppe, einer Cycloalkylgruppe, einer Arylgruppe und einer Heteroarylgruppe; und
m eine ganze Zahl von mindestens 1 ist.

6. Gemustertes Substrat nach einem der Ansprüche 1 bis 5, wobei die ersten Dendrimerstrukturen Endgruppen mit der folgenden allgemeinen Formel (4) oder (5) aufweisen 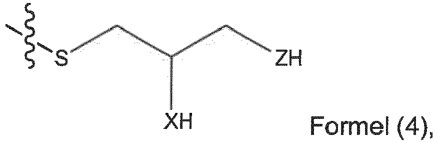
wobei X und Z unabhängig voneinander NR³ oder O sind, wobei jedes R³ unabhängig voneinander ausgewählt ist aus der Gruppe, bestehend aus Wasserstoff, einer Alkylgruppe, einer Cycloalkylgruppe, einer Arylgruppe und einer Heteroarylgruppe; 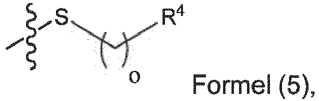
wobei R⁴ ausgewählt ist aus der Gruppe, bestehend aus -NG¹G², -NO₂, -CN, -OG³, -C(O)G⁴, -C(O)NG⁵G⁶, -COOG⁷ und -SO₃G⁸, wobei G¹ bis G³ unabhängig voneinander ausgewählt sind aus der Gruppe, bestehend aus Wasserstoff, einer Alkylgruppe, einer Cycloalkylgruppe, einer Arylgruppe und einer Heteroarylgruppe; und
o eine ganze Zahl von 1 bis 6 ist.

7. Gemustertes Substrat nach einem der Ansprüche 1 bis 6, wobei die zweiten Dendrimerstrukturen Endgruppen mit der folgenden allgemeinen Formel (6) aufweisen 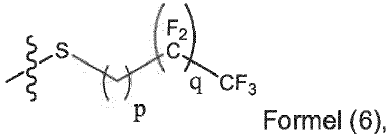
wobei p eine ganze Zahl von 0 bis 10 ist und
q eine ganze Zahl von 3 bis 15 ist.

8. Gemustertes Substrat nach einem der Ansprüche 1 bis 7, wobei die Oberfläche des Substrats, die die ersten und zweiten Bereiche aufweist, ein elektrisch leitfähiges Material aufweist.

9. Verfahren zur Herstellung eines gemusterten Substrats nach einem der Ansprüche 1 bis 8, umfassend das Aufbringen zweiter Dendrimerstrukturen in zweiten Bereichen und erster Dendrimerstrukturen in ersten Bereichen auf einer Oberfläche des Substrats,
wobei die Dendrimerstrukturen jeweils kovalent mit der Substratoberfläche verbunden sind;
die zweiten Bereiche die ersten Bereiche umschließen; und
die zweiten Dendrimerstrukturen mindestens ein Strukturelement aufweisen, das sich von den Strukturelementen der ersten Dendrimerstrukturen unterscheidet

10. Verfahren nach Anspruch 9, umfassend die Schritte
(a) Bereitstellen eines Substrats, das eine Oberfläche umfasst, die Hydroxylgruppen oder Silanolgruppen aufweist;
(b) Umsetzen der Hydroxylgruppen oder Silanolgruppen dieser Oberfläche mit einem Alkenyl-Silan der folgenden allgemeinen Formel (12), um Alkenyl-Silyl-Gruppen auf der Oberfläche zu bilden 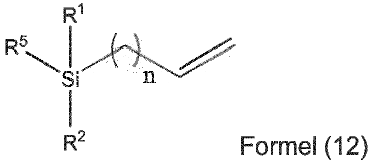
wobei R¹ und R² unabhängig voneinander ausgewählt sind aus der Gruppe, bestehend aus einer Alkylgruppe, einer Cycloalkylgruppe, einer Arylgruppe, einer Heteroarylgruppe, -OA, wobei A unabhängig voneinander ausgewählt ist aus der Gruppe, bestehend aus einer Alkylgruppe, einer Cycloalkylgruppe, einer Arylgruppe und einer Heteroarylgruppe;
R⁵ ein Halogen oder -OQ ist, wobei Q ausgewählt ist aus der Gruppe, bestehend aus einer Alkylgruppe, einer Cycloalkylgruppe, einer Arylgruppe und einer Heteroarylgruppe; und
n eine ganze Zahl von 0 bis 6 ist;
(c) Umsetzen mit einer Thiolverbindung der folgenden allgemeinen Formel (7) 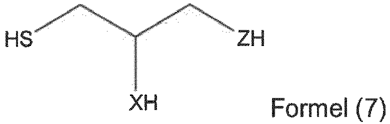 wobei X und Z unabhängig voneinander NR³ oder O sind, wobei jedes R³ unabhängig voneinander ausgewählt ist aus der Gruppe, bestehend aus Wasserstoff, einer Alkylgruppe, einer Cycloalkylgruppe, einer Arylgruppe und einer Heteroarylgruppe;
(d) Umsetzen mit einer Carbonsäure der folgenden allgemeinen Formel (8) oder (9) 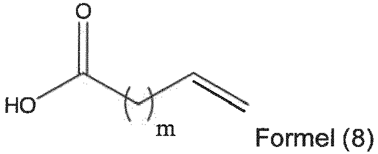 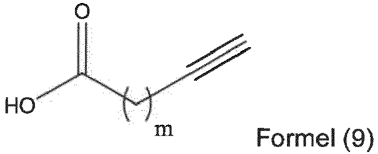 wobei m eine ganze Zahl von mindestens 1 ist;
(e) Mindestens 1-maliges Wiederholen der Schritte (c) und (d) in Kombination;
(f) Gezieltes Umsetzen eines Teiles der Alkenyl- oder Alkinyl-Gruppen, erhalten aus dem letztmaligen Ausführen des Schritts (d), entweder mit
(i) einer Thiolverbindung der folgenden allgemeinen Formel (10), um zweite Dendrimerstrukturen zu erhalten 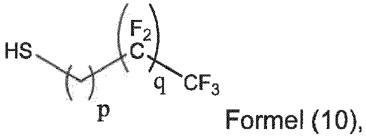
wobei p eine ganze Zahl von 0 bis 10 ist und
q eine ganze Zahl von 3 bis 15 ist; oder mit
(ii) einer Thiolverbindung der allgemeinen Formel (7) oder einer Thiolverbindung der folgenden allgemeinen Formel (11), um erste Dendrimerstrukturen zu erhalten 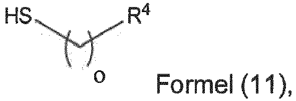
wobei R⁴ ausgewählt ist aus der Gruppe, bestehend aus -NG¹G², -NO₂, -CN, -OG³, -C(O)G⁴, -C(O)NG⁵G⁶, -COOG⁷ und -SO₃G⁸, wobei G¹ bis G⁸ unabhängig voneinander ausgewählt sind aus der Gruppe, bestehend aus Wasserstoff, einer Alkylgruppe, einer Cycloalkylgruppe, einer Arylgruppe und einer Heteroarylgruppe; und
o eine ganze Zahl von 1 bis 6 ist;
(g) Umsetzen der übrigen Alkenyl- oder Alkinyl-Gruppen entweder mit
(i) einer Thiolverbindung der allgemeinen Formel (7) oder einer Thiolverbindung der allgemeinen Formel (11), um erste Dendrimerstrukturen zu erhalten, falls in Schritt (f) zweite Dendrimerstrukturen gebildet wurden; oder mit
(ii) einer Thiolverbindung der allgemeinen Formel (10), um zweite Dendrimerstrukturen zu erhalten, falls in Schritt (f) erste Dendrimerstrukturen gebildet wurden.

11. Verfahren nach Anspruch 10, wobei Schritt (f) die folgenden Schritte (f1) bis (f4) umfasst:
(f1) Aufbringen der entsprechenden Thiolverbindung auf die Oberfläche mit Alkenyl- oder Alkinyl-Gruppen;
(f2) Bedecken der Oberfläche mit der Thiolverbindung mit einer Photomaske;
(f3) Bestrahlen der Oberfläche mit der Thiolverbindung und der Photomaske mit UV-Licht; und
(f4) Entfernen der Photomaske.

12. Verfahren nach Anspruch 10 oder 11, wobei Schritt (g) die folgenden Schritte (g1) und (g2) umfasst:
(g1) Aufbringen der entsprechenden Thiolverbindung auf die Oberfläche mit den übrigen Alkenyl- oder Alkinyl-Gruppen; und
(g2) Bestrahlen der Oberfläche mit der Thiolverbindung mit UV-Licht.

13. Verwendung eines gemusterten Substrats nach einem der Ansprüche 1 bis 8 für die chemische Synthese eines chemischen Syntheseproduktes, als Charakterisierungsplattform und/oder als Plattform zur Zellbehandlung und/oder Zellkultivierung.

14. Verwendung nach Anspruch 13, wobei das gemusterte Substrat zunächst für die chemische Synthese eines chemischen Syntheseproduktes und anschließend als Charakterisierungsplattform zur Charakterisierung des chemischen Syntheseprodukts und/oder für die Behandlung von mindestens einer Zelle mit dem chemischen Syntheseprodukt auf dem gemusterten Substrat verwendet wird.

## Claims

1. A patterned substrate, comprising first regions and second regions on a surface of the substrate, wherein
the first regions have first dendrimer structures and the second regions have second dendrimer structures;
the dendrimer structures are each covalently bonded to the substrate surface; the second regions enclose the first regions;
the second dendrimer structures have at least one structural element that differs from the structural elements of the first dendrimer structures; and
the substrate surface has hydroxyl groups or silanol groups.

2. The patterned substrate according to claim 1, wherein the dendrimer structures have at least two consecutive repeating units and the repeating units each independently have at least two branching points.

3. The patterned substrate according to claim 1 or 2, wherein the dendrimer structures each have an alkylene-silyl group and the dendrimer structures are bonded to the substrate surface by means of the respective alkylene-silyl group.

4. The patterned substrate according to claim 3, wherein the alkylene-silyl group has a structure of the following general formula (1) 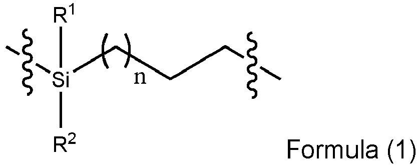
wherein R¹ and R² are independently selected from the group consisting of an alkyl group, a cycloalkyl group, an aryl group, a heteroaryl group, -OA, where A is independently selected from the group consisting of an alkyl group, a cycloalkyl group, an aryl group and a heteroaryl group; and
n is an integer of 0 to 6.

5. The patterned substrate according to one of claims 2 to 4, wherein the first dendrimer structures and the second dendrimer structures each have repeating units having the following general formula (2) or (3) 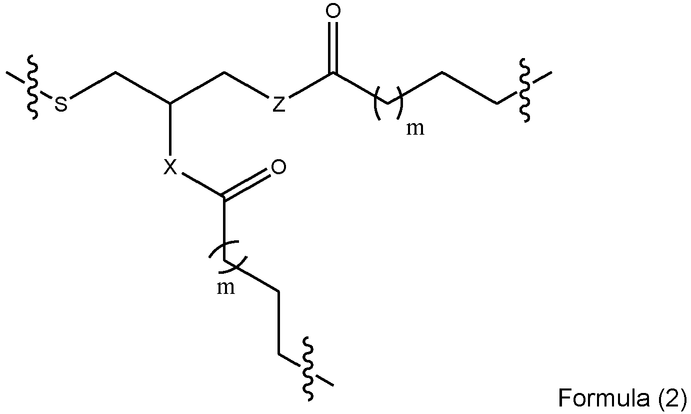 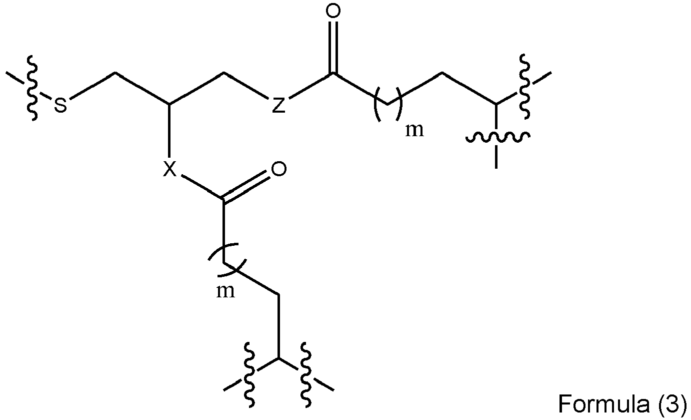
wherein X and Z are independently NR³ or O, each R³ being independently selected from the group consisting of hydrogen, an alkyl group, a cycloalkyl group, an aryl group and a heteroaryl group; and
m is an integer of at least 1.

6. The patterned substrate according to one of claims 1 to 5, wherein the first dendrimer structures have end groups having the following general formula (4) or (5) 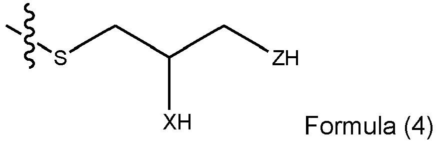
wherein X and Z are independently NR³ or O, each R³ being independently selected from the group consisting of hydrogen, an alkyl group, a cycloalkyl group, an aryl group and a heteroaryl group; 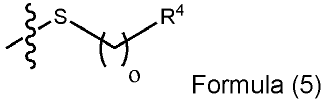
wherein R⁴ is selected from the group consisting of -NG¹G², -NO₂, -CN, -OG³, -C(O)G⁴, -C(O)NG⁵G⁶, -COOG⁷ und -SO₃G⁸, wherein G¹ to G⁸ are independently selected from the group consisting of hydrogen, an alkyl group, a cycloalkyl group, an aryl group and a heteroaryl group; and
o is an integer of 1 to 6.

7. The patterned substrate according to one of claims 1 to 6, wherein the second dendrimer structures have end groups with the following general formula (6) 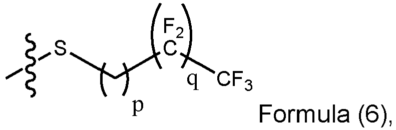
where p is an integer from 0 to 10 and
q is an integer from 3 to 15.

8. The patterned substrate according to one of claims 1 to 7, wherein the surface of the substrate having the first and second regions comprises an electrically conductive material.

9. A method for producing a patterned substrate according to one of claims 1 to 8, comprising applying second dendrimer structures in second regions and first dendrimer structures in first regions on a surface of the substrate,
wherein the dendrimer structures are each covalently bonded to the substrate surface;
the second regions enclose the first regions; and
the second dendrimer structures have at least one structural element that differs from the structural elements of the first dendrimer structures.

10. The method according to claim 9, comprising the steps of
(a) providing a substrate comprising a surface having hydroxyl groups or silanol groups;
(b) reacting the hydroxyl groups or silanol groups of said surface with an alkenyl silane of the following general formula (12) to form alkenyl silyl groups on the surface 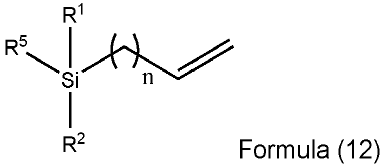
wherein R¹ and R² are independently selected from the group consisting of an alkyl group, a cycloalkyl group, an aryl group, a heteroaryl group, -OA, wherein A is independently selected from the group consisting of an alkyl group, a cycloalkyl group, an aryl group and a heteroaryl group;
R⁵ is a halogen or -OQ, wherein Q is selected from the group consisting of an alkyl group, a cycloalkyl group, an aryl group and a heteroaryl group; and
n is an integer from 0 to 6;
(c) reacting with a thiol compound of the following general formula (7) 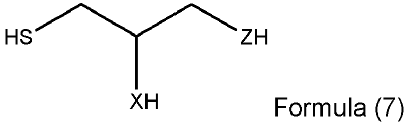 wherein X and Z are independently NR³ or O, each R3 being independently selected from the group consisting of hydrogen, an alkyl group, a cycloalkyl group, an aryl group and a heteroaryl group;
(d) reacting with a carboxylic acid of the following general formula (8) or (9) 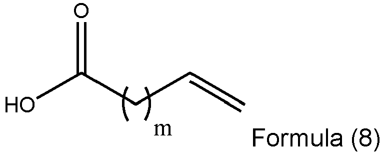 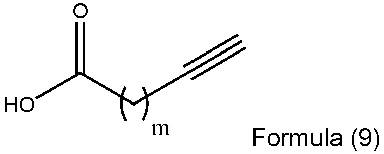 wherein m is an integer of at least 1;
(e) repeating steps (c) and (d) in combination at least once;
(f) targetedly reacting a portion of the alkenyl or alkynyl groups obtained from the last execution of step (d) with either
(i) a thiol compound of the following general formula (10) to obtain second dendrimer structures 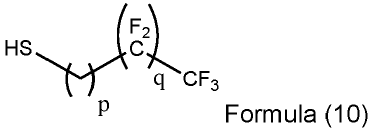
wherein p is an integer from 0 to 10 and
q is an integer from 3 to 15; or with
(ii) a thiol compound of the general formula (7) or a thiol compound of the following general formula (11) to obtain first dendrimer structures 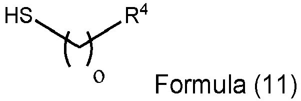
wherein R⁴ is selected from the group consisting of -NG¹G², -NO₂, -CN, -OG³, -C(O)G⁴, -C(O)NG⁵G⁶, -COOG⁷ und -SO₃G⁸, where G¹ to G⁸ are independently selected from the group consisting of hydrogen, an alkyl group, a cycloalkyl group, an aryl group and a heteroaryl group; and
o is an integer from 1 to 6;
(g) reacting the remaining alkenyl or alkynyl groups with either
(i) a thiol compound of the general formula (7) or a thiol compound of the general formula (11) to obtain first dendrimer structures if second dendrimer structures were formed in step (f); or with
(ii) a thiol compound of the general formula (10) to obtain second dendrimer structures if first dendrimer structures were formed in step (f).

11. The method according to claim 10, wherein step (f) comprises the following steps (f1) to (f4):
(f1) applying the corresponding thiol compound to the surface with alkenyl or alkynyl groups;
(f2) covering the surface with the thiol compound with a photomask;
(f3) irradiating the surface with the thiol compound and the photomask with UV light; and
(f4) removing the photomask.

12. The method according to claim 10 or 11, wherein step (g) comprises the following steps (g1) and (g2):
(g1) applying the corresponding thiol compound to the surface with the remaining alkenyl or alkynyl groups; and
(g2) irradiating the surface with the thiol compound with UV light.

13. A use of a patterned substrate according to one of claims 1 to 8 for the chemical synthesis of a chemical synthesis product, as a characterization platform and/or as a platform for cell treatment and/or cell cultivation.

14. The use according to claim 13, wherein the patterned substrate is first used for the chemical synthesis of a chemical synthesis product and then as a characterization platform for characterizing the chemical synthesis product and/or for treating at least one cell with the chemical synthesis product on the patterned substrate.

## Revendications

1. Substrat à motifs comprenant des premières régions et des secondes régions sur une surface du substrat, dans lequel
les premières régions présentent des premières structures de dendrimère et les secondes régions des secondes structures de dendrimère ;
les structures de dendrimère sont chacune reliées par covalence à la surface de substrat ;
les secondes régions entourent les premières régions ;
les secondes structures de dendrimère présentent au moins un élément structurel qui se différencie des éléments structurels des premières structures de dendrimère ; et
la surface de substrat présente des groupes hydroxyle ou groupes silanol.

2. Substrat à motifs selon la revendication 1, dans lequel les structures de dendrimère présentent au moins deux unités répétitives successives et les unités répétitives présentent chacune indépendamment l'une de l'autre au moins deux points de ramification.

3. Substrat à motifs selon la revendication 1 ou 2, dans lequel les structures de dendrimère présentent chacune un groupe alkylène-silyle et les structures de dendrimère sont reliées au moyen du groupe alkylène-silyle respectif à la surface de substrat.

4. Substrat à motifs selon la revendication 3, dans lequel le groupe alkylène-silyle présente une structure de la formule générale suivante (1) 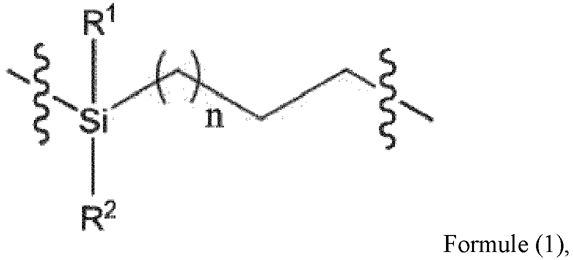
dans laquelle R¹ et R² sont sélectionnés indépendamment l'un de l'autre parmi le groupe constitué d'un groupe alkyle, d'un groupe cycloalkyle, d'un groupe aryle, d'un groupe hétéroaryle, -OA, dans laquelle A est sélectionné indépendamment l'un de l'autre parmi le groupe constitué d'un groupe alkyle, d'un groupe cycloalkyle, d'un groupe aryle et d'un groupe hétéroaryle ; et
n est un nombre entier de 0 à 6.

5. Substrat à motifs selon une des revendications 2 à 4, dans lequel les premières structures de dendrimère et les secondes structures de dendrimère présentent chacune des unités répétitives avec la formule générale suivante (2) ou (3) 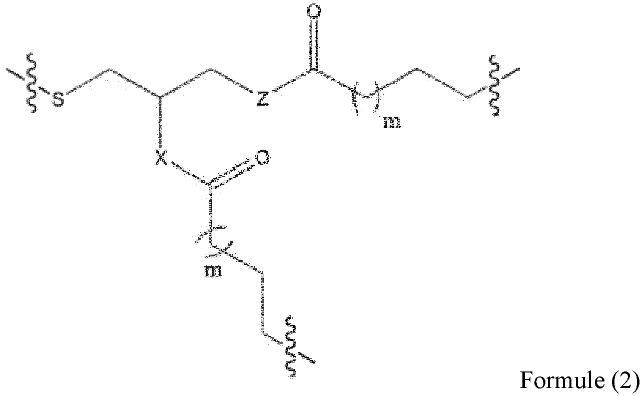 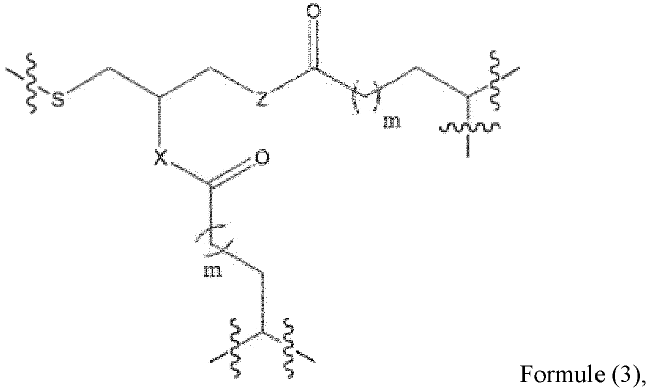 dans lesquelles X et Z sont indépendamment l'un de l'autre NR³ ou O, dans laquelle chaque R³ est sélectionné indépendamment l'un de l'autre parmi le groupe constitué d'hydrogène, d'un groupe alkyle, d'un groupe cycloalkyle, d'un groupe aryle et d'un groupe hétéroaryle ; et
m est un nombre entier d'au moins 1.

6. Substrat à motifs selon une des revendications 1 à 5, dans lequel les premières structures de dendrimère présentent des groupes terminaux avec la formule générale suivante (4) ou (5) 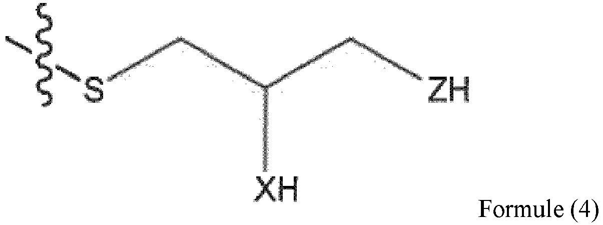
dans laquelle X et Z sont indépendamment l'un de l'autre NR³ ou O, dans laquelle chaque R³ est sélectionné indépendamment l'un de l'autre parmi le groupe constitué d'hydrogène, d'un groupe alkyle, d'un groupe cycloalkyle, d'un groupe aryle et d'un groupe hétéroaryle ; 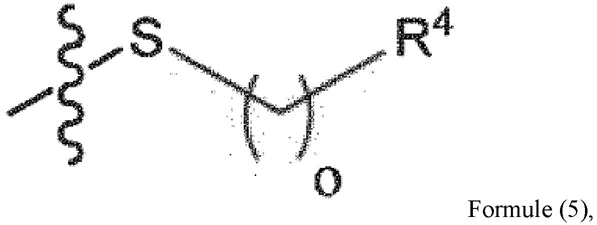
dans laquelle R⁴ est sélectionné parmi le groupe constitué de -NG¹G², -NO₂, - CN, -OG³, -C(O)G⁴, -C(O)NG⁵G⁶, -COOG⁷ et -SO₃G⁸, dans lesquelles G¹ à G⁸ sont sélectionnés indépendamment l'un de l'autre parmi le groupe constitué d'hydrogène, d'un groupe alkyle, d'un groupe cycloalkyle, d'un groupe aryle et d'un groupe hétéroaryle ; et
o est un nombre entier de 1 à 6.

7. Substrat à motifs selon une des revendications 1 à 6, dans lequel les secondes structures de dendrimère présentent des groupes terminaux avec la formule générale suivante (6) 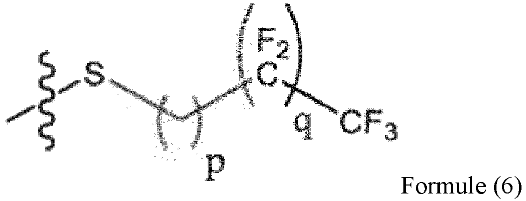
dans laquelle p est un nombre entier de 0 à 10 et
q est un nombre entier de 3 à 15.

8. Substrat à motifs selon une des revendications 1 à 7, dans lequel la surface du substrat qui présente les premières et secondes régions présente un matériau électriquement conducteur.

9. Procédé de fabrication d'un substrat à motifs selon une des revendications 1 à 8, comprenant l'application de secondes structures de dendrimère dans des secondes régions et de premières structures de dendrimère dans des premières régions sur une surface du substrat,
dans lequel les structures de dendrimère sont chacune reliées par covalence à la surface de substrat ;
les secondes régions entourent les premières régions ; et
les secondes structures de dendrimère présentent au moins un élément structurel qui se différencie des éléments structurels des premières structures de dendrimère.

10. Procédé selon la revendication 9, comprenant les étapes de
(a) mise à disposition d'un substrat qui comprend une surface qui présente des groupes hydroxyle ou groupes silanol ;
(b) conversion des groupes hydroxyle ou groupes silanol de cette surface avec un alkényle-silane de la formule générale suivante (12) pour former des groupes alkényle-silyle sur la surface 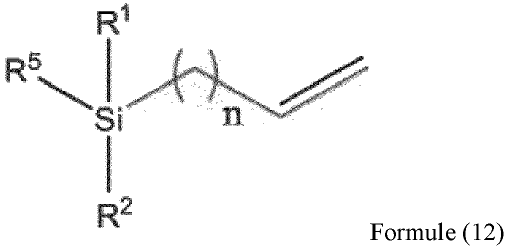
dans laquelle R¹ et R² sont sélectionnés indépendamment l'un de l'autre parmi le groupe constitué d'un groupe alkyle, d'un groupe cycloalkyle, d'un groupe aryle, d'un groupe hétéroaryle, -OA, dans laquelle A est sélectionné indépendamment l'un de l'autre parmi le groupe constitué d'un groupe alkyle, d'un groupe cycloalkyle, d'un groupe aryle et d'un groupe hétéroaryle ;
R⁵ est un halogène ou -OQ, dans laquelle Q est sélectionné parmi le groupe constitué d'un groupe alkyle, d'un groupe cycloalkyle, d'un groupe aryle et d'un groupe hétéroaryle ; et
n est un nombre entier de 0 à 6 ;
(c) conversion avec un composé thiol de la formule générale suivante (7) 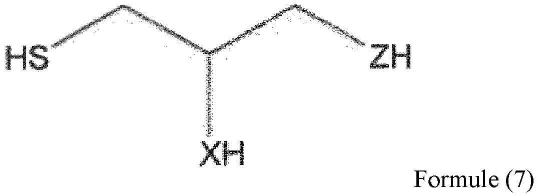 dans laquelle X et Z sont indépendamment l'un de l'autre NR³ ou O, dans laquelle chaque R³ est sélectionné indépendamment l'un de l'autre parmi le groupe constitué d'hydrogène, d'un groupe alkyle, d'un groupe cycloalkyle, d'un groupe aryle et d'un groupe hétéroaryle ;
(d) conversion avec un acide carboxylique de la formule générale suivante (8) ou (9) 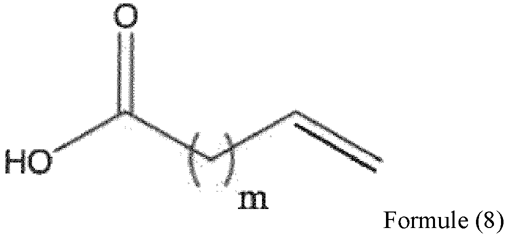 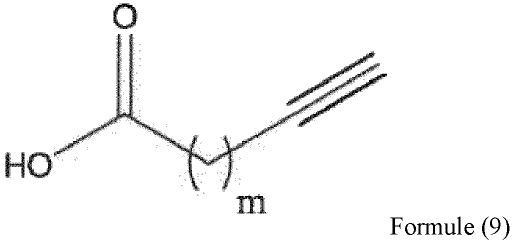 dans lesquelles m est un nombre entier d'au moins 1 ;
(e) répétition au moins 1 fois des étapes (c) et (d) en combinaison ;
(f) conversion ciblée d'une partie des groupes alkényle ou alkynyle, obtenus à partir de la dernière exécution de l'étape (d), soit avec
(i) un composé thiol de la formule générale suivante (10) pour obtenir des secondes structures de dendrimère 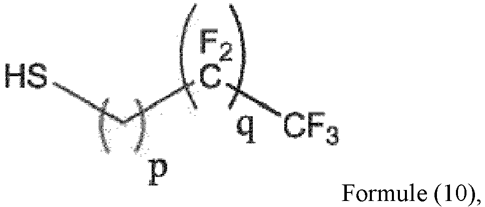
dans laquelle p est un nombre entier de 0 à 10 et
q est un nombre entier de 3 à 15 ; soit avec
(ii) un composé thiol de la formule générale (7) ou un composé thiol de la formule générale suivante (11) pour obtenir des premières structures de dendrimère 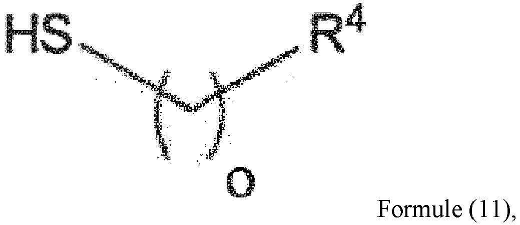
dans laquelle R⁴ est sélectionné parmi le groupe constitué de -NG¹G², -NO₂, - CN, -OG³, -C(O)G⁴, -C(O)NG⁵G⁶, -COOG⁷ et -SO₃G⁸, dans lesquelles G¹ à G⁸ sont sélectionnés indépendamment l'un de l'autre parmi le groupe constitué d'hydrogène, d'un groupe alkyle, d'un groupe cycloalkyle, d'un groupe aryle et d'un groupe hétéroaryle ; et
o est un nombre entier de 1 à 6 ;
(g) conversion des groupes alkényle ou alkynyle restants soit avec
(i) un composé thiol de la formule générale (7) ou un composé thiol de la formule générale (11) pour obtenir des premières structures de dendrimère, au cas où à l'étape (f) des secondes structures de dendrimère ont été formées ; soit avec
(ii) un composé thiol de la formule générale (10) pour obtenir des secondes structures de dendrimère, au cas où à l'étape (f) des premières structures de dendrimère ont été formées.

11. Procédé selon la revendication 10, dans lequel l'étape (f) comprend les étapes suivantes (f1) à (f4) :
(f1) application du composé thiol correspondant sur la surface avec des groupes alkényle ou alkynyle ;
(f2) recouvrement de la surface avec le composé thiol d'un masque photographique ;
(f3) irradiation de la surface avec le composé thiol et du masque photographique avec de la lumière UV ; et
(f4) retrait du masque photographique.

12. Procédé selon la revendication 10 ou 11, dans lequel l'étape (g) comprend les étapes suivantes (g1) et (g2) :
(g1) application du composé thiol correspondant sur la surface avec les groupes alkényle ou alkynyle restants ; et
(g2) irradiation de la surface avec le composé thiol avec de la lumière UV.

13. Utilisation d'un substrat à motifs selon une des revendications 1 à 8 pour la synthèse chimique d'un produit de synthèse chimique, en tant que plate-forme de caractérisation et/ou en tant que plate-forme pour le traitement cellulaire et/ou la culture cellulaire.

14. Utilisation selon la revendication 13, dans laquelle le substrat à motifs est d'abord utilisé pour la synthèse chimique d'un produit de synthèse chimique, puis en tant que plate-forme de caractérisation pour la caractérisation du produit de synthèse chimique et/ou pour le traitement d'au moins une cellule avec le produit de synthèse chimique sur le substrat à motifs.
